# EUROPÄISCHE PATENTSCHRIFT

(11) **EP 1 812 412 B1**
(45) Veröffentlichungstag und Bekanntmachung des Hinweises auf die Patenterteilung: **23.04.2008**
(21) Anmeldenummer: 05806565.7
(22) Anmeldetag: 29.10.2005
(51) Int. Cl.: C07D 277/20, C07D 279/02, C07D 285/00, C07D 285/16, A61K 31/00, A61P 9/00

(54) **SUBSTITUIERTE BENZOYLGUANIDINE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
SUBSTITUTED BENZOYLGUANIDINES METHOD FOR PRODUCTION AND USE THEREOF AS MEDICAMENT OR DIAGNOSTIC AND MEDICAMENT COMPRISING THE SAME
BENZOYLGUANIDINES SUBSTITUEES, PROCEDES POUR LEUR PRODUCTION, LEUR UTILISATION COMME MEDICAMENT OU AGENT DE DIAGNOSTIC ET MEDICAMENT LES CONTENANT

(30) Priorität: 13.11.2004 DE 102004054847
(43) Veröffentlichungstag der Anmeldung: 01.08.2007
(73) Patentinhaber: Sanofi-Aventis Deutschland GmbH, 65929 Frankfurt am Main (DE)
(72) Erfinder: KLEEMAN, Heinz-Werner, 65474 Bischofsheim (DE)
(86) Internationale Anmeldenummer: PCT/EP2005/011595
(87) Internationale Veröffentlichungsnummer: WO 2006/050830

(56) Entgegenhaltungen:
- WO-A-00/30624
- WO-A-03/106410
- WO-A-20/05082895
- BAUMGARTH M ET AL: "BICYCLIC ACYLGUANIDINE NA+/H+ ANTIPORTER INHIBITORS" JOURNAL OF MEDICINAL CHEMISTRY, AMERICAN CHEMICAL SOCIETY. WASHINGTON, US, Bd. 41, Nr. 19, 1998, Seiten 3736-3747, XP000914595 ISSN: 0022-2623
- WEICHERT A ET AL: "SYNTHESIS OF THE HIGHLY SELECTIVE NA+/H+ EXCHANGE INHIBITORS CARIPORIDE MESILATE AND (3-METHANESULFONYL-4-PIPERIDINO)-BENZOYL)G UAN IDINE METHANESULFONATE" ARZNEIMITTEL FORSCHUNG / DRUG RESEARCH, VERLAG FUER NATURWISSENSCHAFTEN GMBH, AULENDORF, DE, Bd. 47, Nr. 11, 1997, Seiten 1204-1207, XP001154676 ISSN: 0004-4172
- BAUMGARTH M ET AL: "(2-METHYL-5-(METHYLSULFONYL)BENZOYL)GUANI DINE NA+/H+ ANTIPORTER INHIBITORS" JOURNAL OF MEDICINAL CHEMISTRY, AMERICAN CHEMICAL SOCIETY. WASHINGTON, US, Bd. 40, Nr. 13, 1997, Seiten 2017-2034, XP000907364 ISSN: 0022-2623
- KARMAZYN, M.: EXPERT OPIN. THER. PATENTS, Bd. 13, Nr. 9, 2003, Seiten 1411-1425, XP002362263

## Beschreibung

### Substituierte Benzoylguanidine der Formel I

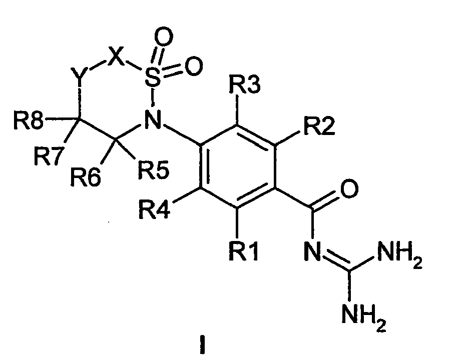

worin R1 bis R8 sowie X und Y die unten angegebenen Bedeutungen haben, und deren pharmazeutisch verträgliche Salze sind substituierte Acylguanidine und inhibieren den zellulären Natrium-Protonen-Antiporter (Na⁺/H⁺-Exchanger, NHE). Aufgrund der NHE-inhibitorischen Eigenschaften eignen sich die Verbindungen der Formel I und deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.

WO 00/30624 offenbart strukturell verwandte Verbindungen zur Behandlung von Diabetes Mellitus.

Gegenüber den bekannten Verbindungen zeichnen sich die erfindungsgemäßen Verbindungen durch eine außerordentlich hohe Wirksamkeit in der Inhibition des Na⁺/H⁺-Austauschs, sowie durch verbesserte ADMET-Eigenschaften aus.

Durch die erfindungsgemäße Struktur wird die Gewebeverteilung vorteilhaft beeinflusst. Das führt u.a. zu erhöhten Expositionen in vivo. Dabei wird das Resorptionsverhalten nicht signifikant beeinflusst und die hohe Bioverfügbarkeit der Acylguanidine bleibt erhalten.

Im Gegensatz zu einigen in der Literatur beschriebenen Acylguanidinen zeigen die hier beschriebenen Verbindungen der Formel I und deren pharmazeutisch verträgliche Salze keine unerwünschten und nachteiligen saliduretischen Eigenschaften.

Die Erfindung betrifft substituierte Benzoylguanidine der Formel 1 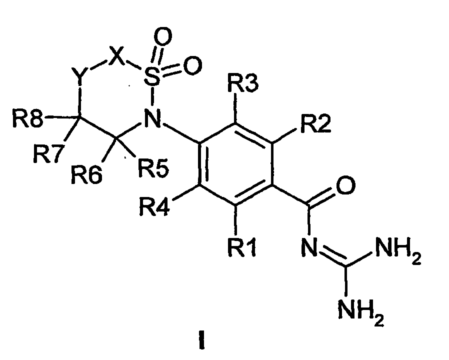 worin bedeuten
- R1: Wasserstoff, Alkyl mit 1, 2, 3 oder 4 C-Atomen, Alkoxy mit 1, 2, 3 oder 4 C-Atomen, F, Cl, Br, I, -CN, NR13R14, -O-(CH₂)ₙ-(CF₂)ₒ-CF₃ oder -(SOₘ)_{q}-(CH₂)ᵣ-(CF₂)ₛ-CF₃;
R13 und R14 unabhängig voneinander Wasserstoff, Alkyl mit 1, 2, 3 oder 4 C-Atomen oder -CH₂-CF₃;
m Null, 1 oder 2
n, o, q, r und s unabhängig voneinander Null oder 1;
- R2: Wasserstoff, Alkyl mit 1, 2, 3 oder 4 C-Atomen, Alkoxy mit 1, 2, 3 oder 4 C-Atomen, F, Cl, Br, I, -CN, NR15R16, -O-(CH₂)ᵤ-(CF₂)ᵥ-CF₃ oder -(SO_{w})ₓ-(CH₂)_{y}-(CF₂)_{z}-CF₃;
R15 und R16 unabhängig voneinander Wasserstoff, Alkyl mit 1, 2, 3 oder 4 C-Atomen oder -CH₂-CF₃;
w Null, 1 oder 2
u, v, x, y und z unabhängig voneinander Null oder 1;
- R3: Wasserstoff, F, Cl, Br, I, -CN, -SO₂CH₃, Alkoxy mit 1, 2, 3 oder 4 C-Atomen, NR9R10, -O-(CH₂)_{b}-(CF₂)_{c}-CF₃, -(SO_{d})ₑ-(CH₂)_{f}-(CF₂)_{g}-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
b, c, e und g unabhängig voneinander Null oder 1;
d Null, 1 oder 2;
f Null, 1, 2, 3 oder 4; oder
- R3: -(CH₂)ₕ -Phenyl oder -O-Phenyl,
in denen die Phenylreste unsubstituiert sind oder substituiert sind mit 1, 2 oder 3 Resten ausgewählt aus der Gruppe bestehend aus Reihe F, Cl, Br, 1, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
j Null oder 1;
k Null, 1, 2 oder 3;
h Null, 1, 2, 3 oder 4; oder
- R3: -(CH₂)ₐₐ -Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1;
cc Null, 1, 2 oder 3;
aa Null, 1, 2, 3 oder 4;
- R4: Wasserstoff, F, Cl, Br, I, -CN, -SO₂CH₃, Alkoxy mit 1, 2, 3 oder 4 C-Atomen, NR11R12, -O-(CH₂)ₑₑ-(CF₂)_{ff}-CF₃, -(SO_{gg})ₕₕ-(CH₂)ⱼⱼ-(CF₂)ₖₖ-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R11 und R12 unabhängig voneinander Wasserstoff, Alkyl mit 1, 2, 3 oder 4 C-Atomen oder -CH₂-CF₃;
ee, ff, hh und kk unabhängig voneinander Null oder 1;
gg Null, 1 oder 2;
jj Null, 1, 2, 3 oder 4; oder
- R4: -(CH₂)ₗₗ -Phenyl oder -O-Phenyl,
in denen die Phenylreste unsubstituiert sind oder substituiert sind mit 1, 2 oder 3 Resten ausgewählt aus der Gruppe bestehend aus Reihe F, Cl, Br, I, -Oₘₘ-(CH₂)ₙₙ-CF₃, Alkoxy mit 1, 2, 3 oder 4 C-Atomen, Alkyl mit 1, 2, 3 oder 4 C-Atomen und -SO₂CH₃;
mm Null oder 1;
nn Null, 1, 2 oder 3;
II Null, 1, 2, 3 oder 4; oder
- R4: -(CH₂)ₒₒ -Heteroaryl,
das unsubstituiert ist oder substituiert ist mit 1, 2 oder 3 Resten ausgewählt aus der Gruppe bestehend aus F, Cl, Br, I, -Opp-(CH₂)ᵣᵣ-CF₃, Alkoxy mit 1, 2, 3 oder 4 C-Atomen, Alkyl mit 1, 2, 3 oder 4 C-Atomen und -SO₂CH₃;
pp Null oder 1;
rr Null, 1, 2 oder 3;
oo Null, 1, 2, 3 oder 4;
- R5 und R6: unabhängig voneinander Wasserstoff oder Alkyl mit 1, 2, 3 oder 4 C-Atomen;
- R7 und R8: unabhängig voneinander Wasserstoff, Alkyl mit 1, 2, 3 oder 4 C-Atomen, Cycloalkyl mit 3, 4, 5, 6, 7 oder 8 C-Atomen, -CF₃ oder Phenyl, Wobei der Phenylrest unsubstituiert ist oder substituiert ist mit 1, 2 oder 3 Resten ausgewählt aus der Gruppe bestehend aus Reihe F, Cl, Br, I, -Oₛₛ-(CH₂)ₜₜ-CF₃, Alkoxy mit 1, 2, 3 oder 4 C-Atomen, Alkyl mit 1, 2, 3 oder 4 C-Atomen und -SO₂CH₃;
ss Null oder 1
tt Null, 1, 2 oder 3; oder
- R7 und R8: bilden zusammen mit dem sie tragenden C-Atom ein Cycloalkyl mit 3, 4, 5, 6, 7 oder 8 C-Atomen;
- X: -CH₂- oder-NR17-
R17 Wasserstoff, Alkyl mit 1, 2, 3 oder 4 C-Atomen, Cycloalkyl mit 3, 4, 5, 6, 7 oder 8 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, I, -Oᵤᵤ-(CH₂)ᵥᵥ-CF₃, Alkoxy mit 1, 2, 3 oder 4 C-Atomen, Alkyl mit 1, 2, 3 oder 4 C-Atomen und -SO₂CH₃;
uu Null oder 1;
w Null, 1, 2 oder 3;
- Y: eine Bindung oder eine Alkylenkette mit 1, 2 oder 3 C-Atomen;
sowie deren pharmazeutisch verträgliche Salze.

Bevorzugt sind Verbindungen der Formel I, in denen bedeuten:
- R1: Wasserstoff, Alkyl mit 1, 2, 3 oder 4 C-Atomen, Alkoxy mit 1, 2, 3 oder 4 C Atomen, F, Cl, NR₁₃R₁₄, -O-(CH₂)ₙ-(CF₂)ₒ-CF₃ oder -(SOₘ)_{q}-(CH₂)ᵣ-(CF₂)ₛ-CF₃;
R13 und R14 unabhängig voneinander Wasserstoff, Methyl, Ethyl oder -CH₂-CF₃;
m Null, 1 oder 2
n, o, q, r und s unabhängig voneinander Null oder 1;
- R2: Wasserstoff, Methyl, Methoxy, F, Cl, -O-CF₃, -O-CH₂-CF₃ oder -S-CF₃;
- R3: Wasserstoff, F, Cl, -CN, -SO₂CH₃, Methoxy, Ethoxy, NR9R10, -O-CF₃, -O-CH₂-CF₃, -SO₂CF₃, -S-CF₃, -CF₃, Alkyl mit 1, 2, 3 oder 4 C-Atomen oder Cycloalkyl mit 3, 4, 5 oder 6 C-Atomen, in dem 1, 2, 3 oder 4 Wasserstoff-Atome durch Fluoratome ersetzt sein können;
R9 und R10 unabhängig voneinander Wasserstoff, Methyl, Ethyl oder -CH₂-CF₃; oder
- R3: Phenyl oder -O-Phenyl,
in denen die Phenylreste unsubstituiert sind oder substituiert sind mit 1, 2 oder 3 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
- R4: Wasserstoff, F, Cl, -CN, -SO₂CH₃ oder Methyl;
- R5 und R6: unabhängig voneinander Wasserstoff, Methyl oder Ethyl;
- R7 und R8: unabhängig voneinander Wasserstoff, Alkyl mit 1, 2, 3 oder 4 C-Atomen, Cycloalkyl mit 3, 4, 5 oder 6 C-Atomen, -CF₃ oder Phenyl, wobei der Phenylrest unsubstituiert ist oder substituiert ist mit 1, 2 oder 3 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
oder
- R7 und R8: bilden zusammen mit dem sie tragenden C-Atom ein Cycloalkyl mit 3, 4, 5 oder 6 C-Atomen;
- X: -CH₂- oder -NR17-
R17 Wasserstoff, Methyl, Ethyl, Cycloalkyl mit 3, 4, 5 oder 6 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, 1, -O-CF₃, -O-CH₂-CF₃, -S-CF₃, Methoxy, Ethoxy, Alkyl mit 1, 2, 3 oder 4 C-Atomen und -SO₂CH₃;
- Y: eine Bindung oder -CH₂-;
sowie deren pharmazeutisch verträgliche Salze.

Besonders bevorzugt sind Verbindungen der Formel I, in denen bedeuten:
- R1: Wasserstoff, Methyl, Ethyl, Methoxy, Ethoxy, F, Cl, -O-CF₃, -O-CH₂-CF₃ oder -S-CF₃;
- R2: Wasserstoff, F, Cl, -O-CF₃, -O-CH₂-CF₃ oder -S-CF₃;
- R3: F, Cl, -CN, -SO₂CH₃, -CF₃, -O-CF₃, -O-CH₂-CF₃, -SO₂CF₃, -S-CF₃, Alkyl mit 1, 2, 3 oder 4 C-Atomen oder Cycloalkyl mit 3, 4, 5 oder 6 C-Atomen, in dem 1, 2, 3 oder 4 Wasserstoff-Atome durch Fluoratome ersetzt sein können;
oder
- R3: Phenyl oder -O-Phenyl,
in denen die Phenylreste unsubstituiert sind oder substituiert sind mit 1oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
- R4: Wasserstoff oder F;
- R5 und R6: unabhängig voneinander Wasserstoff oder Methyl;
- R7 und R8: unabhängig voneinander Wasserstoff, Alkyl mit 1, 2, 3 oder 4 C-Atomen, Cycloalkyl mit 3, 4, 5 oder 6 C-Atomen, -CF₃ oder Phenyl, wobei der Phenylrest unsubstituiert ist oder substituiert ist mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
oder
- R7 und R8: bilden zusammen mit dem sie tragenden C-Atom ein Cycloalkyl mit 3, 4, 5 oder 6 C-Atomen;
- X: -CH₂- oder -NR17-
R17 Wasserstoff, Methyl, Ethyl, Cycloalkyl mit 3, 4, 5 oder 6 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, I, -O-CF₃, -O-CH₂-CF₃, -S-CF₃, Methoxy, Ethoxy, Alkyl mit 1, 2, 3 oder 4 C-Atomen und -SO₂CH₃;
- Y: eine Bindung oder -CH₂-;
sowie deren pharmazeutisch verträgliche Salze.

Ganz besonders bevorzugt sind Verbindungen der Formel 1, in denen bedeuten:
- R1: Wasserstoff, Methyl -O-CH₂-CF₃, oder -S-CF₃;
- R2: Wasserstoff;
- R3: -CN, -SO₂CH₃, -CF₃, -O-CF₃, -O-CH₂-CF₃, -SO₂CF₃, -S-CF₃;
- R4: Wasserstoff;
- R5 und R6: Wasserstoff;
- R7: Wasserstoff;
- R8: Alkyl mit 1, 2, 3 oder 4 C-Atomen, Cycloalkyl mit 3, 4, 5 oder 6 C-Atomen, -CF₃ oder Phenyl, wobei der Phenylrest unsubstituiert ist oder substituiert ist mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
- X: -CH₂-
- Y: eine Bindung oder -CH₂-;
sowie deren pharmazeutisch verträgliche Salze.

Ebenfalls ganz besonders bevorzugt sind Verbindungen der Formel I, in denen bedeuten:
- R1: Wasserstoff, Methyl -O-CH₂-CF₃, oder -S-CF₃;
- R2: Wasserstoff;
- R3: -CN, -SO₂CH₃, -CF₃, -O-CF₃, -O-CH₂-CF₃, -SO₂CF₃, -S-CF₃;
- R4: Wasserstoff;
- R5 und R6: unabhängig voneinander Wasserstoff oder Methyl;
- R7: Wasserstoff;
- R8: Wasserstoff,
- X: -CH₂- oder -NR17-
R17 Wasserstoff, Methyl, Ethyl, Cycloalkyl mit 3, 4, 5 oder 6 C-Atomen oder -CH₂-CF₃;
Y eine Bindung oder -CH₂-;
sowie deren pharmazeutisch verträgliche Salze.

In einer Ausführungsform werden Verbindungen der Formel I bevorzugt, in denen R1 1 durch Wasserstoff, Alkyl mit 1, 2, 3 oder 4 C-Atomen, Alkoxy mit 1, 2, 3 oder 4 C Atomen, F, Cl, NR13R14, -O-(CH₂)ₙ-(CF₂)ₒ-CF₃ oder -(SOₘ)_{q}-(CH₂)ᵣ-(CF₂)ₛ-CF₃ beschrieben wird, wobei R13 und R14 unabhängig voneinander Wasserstoff, Methyl, Ethyl oder -CH₂-CF₃ sind, m Null, 1 oder 2 ist und n, o, q, r und s unabhängig voneinander Null oder 1 sind; besonders bevorzugt sind Verbindungen der Formel 1, in denen R1 durch Wasserstoff, Methyl, Ethyl, Methoxy, Ethoxy, F, Cl, -O-CF₃, -O-CH₂-CF₃ oder -S-CFg, insbesondere Wasserstoff, Methyl -O-CH₂-CF₃, oder -S-CF₃, beschrieben wird. Speziell bevorzugt sind Verbindungen, in denen R1 durch Wasserstoff oder Methyl beschrieben wird.

In einer weiteren Ausführungsform sind Verbindungen der Formel I bevorzugt, in denen R2 durch Wasserstoff, Methyl, Methoxy, F, Cl, -O-CF₃, -O-CH₂-CF₃ oder -S-CF₃ , insbesondere Wasserstoff, F, Cl, -O-CF₃, -O-CH₂-CF₃ oder -S-CF₃, beschrieben wird, besonders bevorzugt sind Verbindungen, in denen R2 durch Wasserstoff beschrieben wird.

In einer weiteren Ausführungsform sind Verbindungen der Formel I bevorzugt, in denen R3 durch Wasserstoff, F, Cl, -CN, -SO₂CH₃, Methoxy, Ethoxy, NR9R10, -O-CF₃, -O-CH₂-CF₃, -SO₂CF₃, -S-CF₃, Alkyl mit 1, 2, 3 oder 4 C-Atomen oder Cycloalkyl mit 3, 4, 5 oder 6 C-Atomen, in dem 1, 2, 3 oder 4 Wasserstoff-Atome durch Fluoratome ersetzt sein können, wobei R9 und R10 unabhängig voneinander Wasserstoff, Methyl, Ethyl oder -CH₂-CF₃ sind, oder durch Phenyl oder -O-Phenyl beschrieben wird, in denen die Phenylreste unsubstituiert sind oder substituiert sind mit 1, 2 oder 3 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -O₂CH₃;besonders bevorzugt sind Verbindungen, in denen R3 durch F, Cl, -CN, -O₂CH₃, -CF₃, -O-CF₃, -O-CH₂-CF₃, -SO₂CF₃, -S-CF₃, Alkyl mit 1, 2, 3 oder 4 C-Atomen oder Cycloalkyl mit 3, 4, 5 oder 6 C-Atomen, in dem 1, 2, 3 oder 4 Wasserstoff-Atome durch Fluoratome ersetzt sein können;
Oder durch Phenyl oder -O-Phenyl beschrieben wird, in denen die Phenylreste unsubstituiert sind oder substituiert sind mit 1oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃; speziell bevorzugt sind Verbindungen, in denen R3 durch -CN, -SO₂CH₃, -CF₃, -O-CF₃, -O-CH₂-CF₃, -O₂CF₃, -S-CF₃, insbesondere -SO₂CH₃ oder -CF₃, beschrieben wird.

In einer weiteren Ausführungsform sind Verbindungen der Formel I bevorzugt, in denen R4 durch Wasserstoff, F, Cl, -CN, -SO₂CH₃ oder Methyl, insbesondere Wasserstoff oder F, beschrieben wird, besonders bevorzugt sind Verbindungen, in denen R4 durch Wasserstoff beschrieben wird.

In einer weiteren Ausführungsform sind Verbindungen der Formel I bevorzugt, in denen R5 und R6 unabhängig voneinander durch Wasserstoff, Methyl oder Ethyl beschrieben wird; besonders bevorzugt sind Verbindungen, in denen R5 und R6 unabhängig voneinander durch Wasserstoff oder Methyl, beispielsweise Wasserstoff, beschrieben werden.

In einer weiteren Ausführungsform sind Verbindungen der Formel I bevorzugt, in denen R7 und R8 unabhängig voneinander durch Wasserstoff, Alkyl mit 1, 2, 3 oder 4 C-Atomen, Cycloalkyl mit 3, 4, 5 oder 6 C-Atomen, -CF₃ oder Phenyl beschrieben werden, wobei der Phenylrest unsubstituiert ist oder substituiert ist mit 1, 2 oder 3 Resten, beispielsweise mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃, oderin denen R7 und R8 zusammen mitdem sie tragenden C-Atom ein Cycloalkyl mit 3, 4, 5 oder 6 C-Atomen bilden; besonders bevorzugt sind Verbindungen, in denen R7 durch Wasserstoff und R8 durch Alkyl mit 1, 2, 3 oder 4 C-Atomen, Cycloalkyl mit 3, 4, 5 oder 6 C-Atomen, -CF₃ oder Phenyl beschrieben wird, wobei der Phenylrest unsubstituiert ist oder substituiert ist mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃; beispielsweise wird R8 durch Phenyl beschrieben, das unsubstituiert ist oder unsubstituiert ist mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃, insbesondere F. In einer weiteren Ausführungsform sind Verbindungen bevorzugt, in denen R7 und R8 Wasserstoff sind.

In einer weiteren Ausführungsform sind Verbindungen der Formel I bevorzugt, in denen X durch -CH₂- oder-NR17- beschrieben wird, wobei R17 Wasserstoff, Methyl, Ethyl, Cycloalkyl mit 3, 4, 5 oder 6 C-Atomen, -CH₂-CF₃ oder Phenyl ist, das unsubstituiert ist oder substituiert ist mit 1, 2 oder 3 Resten ausgewählt aus der Gruppe bestehend aus F, Cl, Br, I, -O-CF₃, -O-CH₂-CF₃, -S-CF₃, Methoxy, Ethoxy, Alkyl mit 1, 2, 3 oder 4 C-Atomen und -SO₂CH₃; bevorzugt ist R17 Wasserstoff, Methyl, Ethyl, Cycloalkyl mit 3, 4, 5 oder 6 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, I, -O-CF₃, -O-CH₂-CF₃, -S-CF₃, Methoxy, Ethoxy, Alkyl mit 1, 2, 3 oder 4 C-Atomen und -SO₂CH₃; besonders bevorzugt wird R17 durch Wasserstoff, Methyl, Ethyl, Cycloalkyl mit 3, 4, 5 oder 6 C-Atomen oder -CH₂-CF₃ beschrieben; beispielsweise wird R17 beschrieben durch Cycloalkyl mit 3, 4, 5 oder 6 C-Atomen, zum Beispiel Cyclopropyl. In einer weiteren Ausführungsform ist X bevorzugt -CH₂-.

In einer weiteren Ausführungsform sind Verbindungen der Formel 1 bevorzugt, in denen Y durch eine Bindung oder -CH₂- beschrieben wird.

Speziell bevorzugt sind Verbindungen der Formel I ausgewählt aus der Gruppe:
N-[4-(1,1-Dioxo-isothiazolidin-2-yl)-5-methansulfonyl-2-methyl-benzoyl]-guanidin,
N-[4-(1,1-Dioxo-[1,2]thiazinan-2-yl)-5-methansulfonyl-2-methyl-benzoyl]-guanidin,
N-[4-(3,3-Dimethyl-1,1-dioxo-isothiazolidin-2-yl)-5-methansulfonyl-2-methyl-benzoyl]-guanidin,
N-[4-(1,1-Dioxo-4-phenyl-isothiazolidin-2-yl)-5-methansulfonyl-2-methyl-benzoyl]-guanidin,
N-{4-[4-(4-Fluor-phenyl)-1,1-dioxo-isothiazolidin-2-yl]-5-methansulfonyl-2-methyl-benzoyl}-guanidin,
N-[4-(1,1-Dioxo-4-phenyl-1-[1,2]thiazinan-2-yl)-5-methansulfonyl-2-methyl-benzoyl]-guanidin,
N-[4-(5-Cyclopropyl-1,1-dioxo-1-[1,2,5]thiadiazolidin-2-yl)-5-methansulfonyl-2-methyl-benzoyl]-guanidin,
N-[4-(6-Cyclopropyl-1,1-dioxo-1-[1,2,6]thiadiazinan-2-yl)-5-methansulfonyl-2-methyl-benzoyl]-guanidin,
N-[4-(1,1-Dioxo-1-isothiazolidin-2-yl)-3-trifluoromethyl-benzoyl]-guanidin,
N-[4-(1,1-Dioxo-1-[1,2]thiazinan-2-yl)-3-trifluoromethyl-benzoyl]-guanidin
und deren pharmazeutisch verträgliche Salze.

Enthalten die Substituenten R1 bis R8 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben vorliegen.

Die vorliegende Erfindung umfasst alle tautomeren Formen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl und Isopropyl. In Alkylresten können ein oder mehrere, zum Beispiel 1, 2, 3, 4 oder 5 Wasserstoffatome durch Fluoratome substituiert sein. Beispiele für solche Fluoralkylreste sind Trifluormethyl, 2,2,2-Trifluorethyl und Pentafluorethyl. Substituierte Alkylreste können in beliebigen Positionen substituiert sein.

Beispiele für Cycloalkylreste sind Cyclopropyl, Cyclobutyl, Cyclopentyl, Cyclohexyl Cycloheptyl oder Cyclooctyl. In Cycloalkylresten können ein oder mehrere, zum Beispiel 1, 2, 3, oder 4 Wasserstoffatome durch Fluoratome substituiert sein. Substituierte Cycloalkylreste können in beliebigen Positionen substituiert sein.

Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. Dies gilt ebenso für substituierte Phenylreste in Gruppen wie zum Beispiel Phenylalkyl oder Phenyloxy. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden.

Heteroarylreste sind aromatische Ringverbindungen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über die 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für die Heteroarylreste wie zum Beispiel im Rest Heteroarylalkyl. Heteroaryl bedeutet zum Beispiel Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinyl.

Als Heteroarylreste gelten insbesondere 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder -5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder -5-yl, 1,2,4-Oxadiazol-3- oder -5-yl, 1,3,4-Oxadiazol-2-yl oder-5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder -5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, -3- oder -4-pyridyl.

Besonders bevorzugt sind die Heteroaromaten 2- oder 3-Thienyl, 2- oder 3-Furyl, 1-, 2- oder 3- Pyrrolyl, 1-, 2-, 4- oder 5-Imidazolyl, 2-, 3-, 4-, 5-, 6-, 7- oder 8-Chinolyl, 1-, 3-, 4- oder 5-Pyrazolyl, 2-, 3- oder 4-Pyridyl, 2- oder 3-Pyrazinyl, 2-, 4-, 5- oder 6-Pyrimidinyl und 3- oder 4-Pyridazinyl.

Die Erfindung betrifft weiterhin ein Verfahren zur Herstellung der Verbindungen der Formel I, dadurch gekennzeichnet, dass eine Verbindung der Formel II 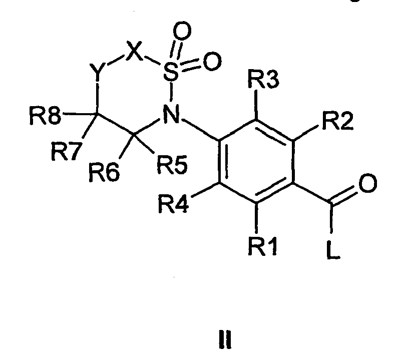 worin R1 bis R8 die Bedeutung wie in Verbindungen der Formel I besitzen und L für eine nucleophil substituierbare Fluchtgruppe steht, mit Guanidin umsetzt wird.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in dem Fachmann bekannter Weise aus den zugrundeliegenden Carbonsäurechloriden (Formel II; L = Cl), die man ihrerseits wiederum in bekannter Weise aus den zugrundeliegenden Carbonsäuren (Formel II; L = OH) beispielsweise mit Thionylchlorid herstellen kann.
Neben den Carbonsäurechloriden der Formel II (L = Cl) lassen sich auch weitere aktivierte Säurederivate der Formel II in bekannter Weise direkt aus den zugrundeliegenden Benzoesäuren (Formel II; L = OH) herstellen, wie die Methylester der Formel II mit L = OCH₃ durch Behandeln mit gasförmigem HCl in Methanol, die Imidazolide der Formel II durch Behandeln mit Carbonyldiimidazol, die gemischten Anhydride der Formel II durch Behandeln mit Cl-COOC₂H₅ oder Tosylchlorid in Gegenwart von Triethylamin in einem inerten Lösungsmittel, wie auch Aktivierungen von Benzoesäuren mit Dicyclohexylcarbodümid (DCC) oder mit O-[(Cyano(ethoxycarbonyl)methylen)amino]-1,1,3,3-tetramethyluronium-tetrafluorborat ("TOTU") möglich sind. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985, S. 350) angegeben.

Die Umsetzung eines aktivierten Carbonsäurederivates der Formel 11 mit Guanidin erfolgt vorzugsweise in bekannter Weise in einem protischen oder aprotischen polaren aber inerten organischen Lösungsmittel. Dabei haben sich bei der Umsetzung der Benzoesäuremethylester (Formel II; L = OCH₃) mit Guanidin Methanol, Isopropanol oder THF bei Temperaturen von 20°C bis zur Siedetemperatur dieser Lösungsmittel bewährt. Bei den meisten Umsetzungen von Verbindungen der Formel 11 mit salzfreiem Guanidin wird beispielsweise in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von Verbindungen der Formel II mit Guanidin verwendet werden.

Wenn L die Bedeutung Cl hat, arbeitet man vorteilhaft unter Zusatz eines Säurefängers, zum Beispiel in Form von überschüssigem Guanidin, zur Abbindung der Halogenwasserstoffsäure.

In einer Ausführungsform sind Verbindungen der Formel II bevorzugt, in denen L beschrieben wird durch Alkoxy mit 1, 2, 3 oder 4 C-Atomen, Benzyloxy, Phenoxy, Phenylthio, Methylthio, 2-Pyridylthio, Stickstoffheterocyclus, beispielsweise 1-Imidazolyl, F, Cl, Br, I oder OH; besonders bevorzugt sind Verbindungen der Formel II, in denen L beschrieben wird durch Methoxy, Cl oder OH.

Die Erfindung betrifft weiterhin ein Verfahren zur Herstellung der Verbindungen der Formel II, 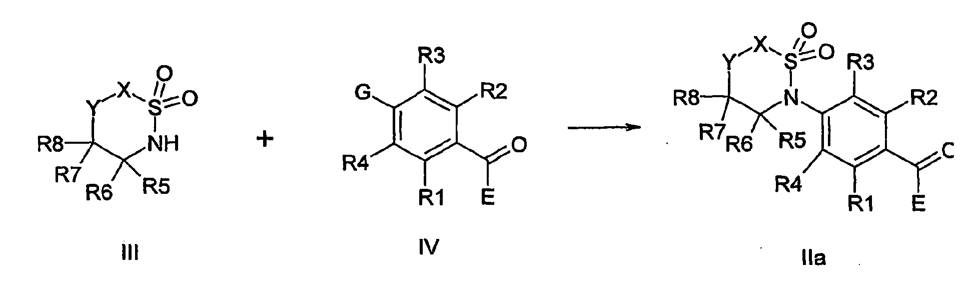 dadurch gekennzeichnet, dass eine Verbindung der Formel III mit einer Verbindung der Formel IV in einer nucleophilen aromatischen Substitution zu einer Verbindung der Formel IIa umgesetzt wird,
worin R1 bis R8, X und Y die angegebene Bedeutung besitzen und
- E: Alkoxy mit 1, 2, 3 oder 4 C-Atomen, Benzyloxy oder Phenoxy;
- G: F, Cl, Br oder 1 bedeuten;

Eine Verbindung der Formel III wird hierbei mit einer Verbindung der Formel IV zusammen mit einer anorganischen Base, bevorzugt K₂CO₃ oder Cs₂CO₃, oder einer organischen Base, bevorzugt TBTMG in einem dipolar aprotischen Lösungsmittel, bevorzugt DMF oder NMP bei einer Temperatur zwischen -40°C und dem Siedepunkt des Lösungsmittels, bevorzugt zwischen 0°C und 140°C zu den Verbindungen der Formel IV umgesetzt.

Gegebenenfalls wird ein Ester der Formel IIa zu Verbindungen der Formel II weiter umbesetzt, beispielsweise zur Säure der Formel II (L = OH) verseift oder nach dem Fachmann bekannten Methoden in ein Säurechlorid der Formel II (L = Cl) überführt.

Sultame und cyclische Schwefelsäureamide der Formel III können analog zu bekannten Methoden synthetisiert (Pharmaceutical Chemistry Journal (Übersetzung von Khimiko-Farmatsevticheskii Zhurnal) (2000), Volume Date 1999, 33(11), 598; J. Org. Chem. (1991), 56, 3549; Tetrahedron 59, (2003) 6051).

Verbindungen der Formel IV können analog zu bekannten Methoden synthetisiert werden, wie beispielsweise beschrieben in J. Chem. Soc., Chem. Commun., 1993, 1359; J. Med. Chem. 1998, 41, 3736; J. Med. Chem. 1997, 40, 2017; Bioorg. Med. Chem. Lett. 13 (2003), 4085; Eur. J. Org. Chem. 2002,1490.

In den Ausgangsverbindungen können auch funktionelle Gruppen in geschützter Form oder in Form von Vorstufen vorliegen und dann in den nach dem oben beschriebenen Verfahren hergestellten Verbindungen der Formel II in die gewünschten Gruppen überführt werden. Entsprechende Schutzgruppentechniken sind dem Fachmann bekannt.
Ebenso können entsprechende funktionelle Gruppen nach dem Fachmann bekannten Methoden derivatisiert werden.

Die Erfindung betrifft weiterhin Verbindungen der Formel II: 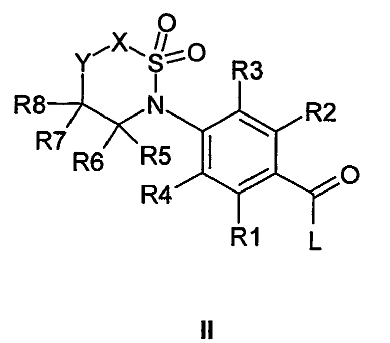 worin R1 bis R8, X und Y wie in Verbindungen der Formel 1 definiert sind und L wie oben beschrieben definiert ist,
wobei Verbindungen der Formel II ausgenommen sind, in denen R1, R2, R3 und R4 gleichzeitig Wasserstoff sind und X -CH₂- ist,
und wobei die folgenden Verbindungen ausgeschlossen sind
4-(1,1-Dioxo-isothiazolidin-2-yl)-3-methyl-benzoesäuremethylester,
4-(1, 1 -Dioxo-isothiazolidin-2-yl)-3-methyl-benzoesäure,
4-(1,1-Dioxo-1-[1,2,6]thiadiazinan-2-yl)-3-methyl-benzoesäuremethylester,
4-(1,1-Dioxo-1-[1,2,6]thiadiazinan-2-yl)-3-methyl-benzoesäure
und
3-Chlor-4-(1,1-Dioxo-1-[1,2]thiazinan-2-yl)-benzoesäure.

Benzoylguanidine der Formel I und Verbindungen der Formel II sind im allgemeinen schwache Basen und können Säure unter Bildung von Salzen binden. Als Säureadditionssalze kommen Salze aller pharmazeutisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen der Formel I sind substituierte Acylguanidine und inhibieren den zellulären Natrium-Protonen-Antiporter (Na⁺/H⁺-Exchanger, NHE), insbesondere den Subtyp NHE-1.
Gegenüber bekannten NHE-Inhibitoren zeichnen sich die erfindungsgemäßen Verbindungen durch eine außerordentlich hohe Wirksamkeit in der Inhibition des Na⁺/H⁺-Austauschs aus, sowie durch verbesserte ADMET-Eigenschaften aus, zum Beispiel durch längere S9-Stabilitäten (Leberstabilitäten, Stabilität gegenüber enzymatischem Angriff) und hoher Selektivität gegenüber dem hERG-Kaliumkanal. Dabei weisen sie ein gutes Resorptionsverhalten und eine hohe Bioverfügbarkeit auf.

Aufgrund der NHE-inhibitorischen Eigenschaften eignen sich die Verbindungen der Formel I und/oder deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.

Da NHE-Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydrasen, Inhibitoren der Bicarbonationen transportierenden Systeme wie des Natrium-Bicarbonat-Cotransporters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der erfindungsgemäßen Verbindungen betrifft die Prävention und die Behandlung akuter und chronischer Krankheiten in der Veterinär- und in der Humanmedizin.

So eignen sich die erfindungsgemäßen Inhibitoren des NHE zur Behandlung von Krankheiten, die durch Ischämie und durch Reperfusion hervorgerufen werden.

Die hier beschriebenen Verbindungen sind infolge ihrer pharmakologischen Eigenschaften als antiarrhythmische Arzneimittel geeignet.
Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I und/oder deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden sind können die erfindungsgemäßen Verbindungen der Formel I auch bei Wiederbelebung nach einem Herzstillstand eingesetzt werden.
Die erfindungsgemäßen Verbindungen sind von Interesse für Arzneimittel gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit Verbindungen der Formel I und/oder deren pharmazeutisch verträgliche Salze geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können die erfindungsgemäßen NHE1-Inhibitoren der Formel I und/oder deren pharmazeutisch verträgliche Salze bei einer herzschädigenden Überproduktion von Schilddrüsenhormonen, der Thyreotoxikose, oder bei der externen Zufuhr von Schilddrüsenhormonen Verwendung finden. Die Verbindungen der Formel I und/oder deren pharmazeutisch verträgliche Salze eignen sich somit zur Verbesserung der Therapie mit cardiotoxischen Arzneimitteln.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet,
wobei sie zum Beispiel zur Behandlung des Schlaganfalls oder des Hirnödems geeignet sind.

Die Verbindungen der Formel I und/oder deren pharmazeutisch verträgliche Salze eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydrasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäß verwendeten Verbindungen der Formel I und/oder deren pharmazeutisch verträgliche Salze ebenfalls zur Behandlung von Formen des Schocks, wie beispielweise des allergischen, cardiogenen, hypovolämischen und des bakteriellen Schocks.

Die Verbindungen der Formel und/oder deren pharmazeutisch verträgliche Salze können ebenfalls zur Prävention und zur Behandlung thrombotischer Erkrankungen verwendet werden, da sie als NHE-Inhibitoren die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und der thrombogenen Selectin-Proteine hemmen bzw. verhindern. Damit kann die pathogene Wirkung bedeutender thrombogener Faktoren vermindert und ausgeschaltet werden.

Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan Rezeptor-Antagonisten, Phosphodiesterase-Inhibitoren, Factor-Vlla-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydrase, wie beispielsweise mit Acetazolamid, ist besonders günstig.

Darüber hinaus zeichnen sich NHEI-Inhibitoren durch starke inhibierende Wirkung auf die Proliferationen von Zellen, beispielsweise der Fibroblasten-Proliferation und der Proliferation der glatten Gefäßmuskelzellen, aus. Deshalb kommen die Verbindungen der Formel I und/oder deren pharmazeutisch verträgliche Salze als wertvolle Therapeutika für Krankheiten in Frage, bei denen die Proliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch NHE-Inhibitoren die Zellmigration inhibiert wird. Daher kommen die Verbindungen der Formel I und/oder deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

NHE1-Inhibitoren zeichnen sich weiterhin durch eine Verzögerung oder Verhinderung von fibrotischen Erkrankungen aus. Verbindungen der Formel 1 und/oder deren pharmazeutisch verträgliche Salze eignen sich somit als Mittel zur Behandlung von Fibrosen des Herzens, sowie der Lungenfibrose, Leberfibrose, der Nierenfibrose und anderer fibrotischer Erkrankungen. Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formel I und/oder deren pharmazeutisch verträgliche Salze zur Prävention und zur Behandlung des Bluthochdrucks und zur Behandlung von Herz-Kreislauferkrankungen. Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromakalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1-Inhibitoren eine signifikante antiphlogistische Wirkung haben und somit als Antiinflammatorika verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet. Weiterhin könne die erfindungsgemäßen Verbindungen zur Prävention oder Behandlung von Erkrankungen, die durch Protozoen verursacht werden, eingesetzt werden, wie bei Malaria und der Hühnercoccidiose.

Es wurde außerdem gefunden, dass NHE1-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, dass NHE1-Inhibitoren bezüglich der Beeinflussung der Serumlipidspiegel wertvolle therapeutisch verwertbare Eigenschaften zeigen. So erniedrigen sie signifikant die erhöhte SerumKonzentrationen von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen die NHE1-Inhibitoren zu einer deutlichen Reduktion der durch Stoffwechselanomalien induzierten Infarkte und insbesondere zu einer signifikanten Verminderung der induzierten Infarktgröße und dessen Schweregrades.

Die Verbindungen der Formel I und/oder deren pharmazeutisch verträgliche Salze finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmem und AngiotensinRezeptorantagonisten. Eine Kombination eines NHE-Inhibitors der Formel I und/oder deren pharmazeutisch verträgliche Salze mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors der Formel I und/oder deren pharmazeutisch verträgliche Salze steigert, erweist sich als günstige Kombination mit verstärkter Wirkung und vermindertem Wirkstoffeinsatz.

So führen Verbindungen der Formel I und/oder deren pharmazeutisch verträgliche Salze zu einen wirksamen Schutz gegen Endothelschädigungen unterschiedlicher Genese. Mit diesem Schutz der Gefäße gegen das Syndrom der endothelialen Dysfunktion sind Verbindungen der Formel I und/oder deren pharmazeutisch verträgliche Salze wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass NHE1-Inhibitoren geeignet in der Behandlung des nicht-insulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR Agonisten, wie Rosiglitazone, Pioglitazone etc., mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken die Verbindungen der Formel I und/oder deren pharmazeutisch verträgliche Salze der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin dürfte dabei eine besondere Bedeutung zukommen.

NHE1-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die unabhängig von akuten Mangeldurchblutungszuständen sind und bei normalen, nichtischämischen Bedingungen auftreten. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. Die Verbindungen der Formel I und/oder deren pharmazeutisch verträgliche Salze eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung des Herzens. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. Die Verbindungen der Formel I und/oder deren pharmazeutisch verträgliche Salze eignen sich somit hervorragend zur Behandlung und Prävention der Herzinsuffizienz, des congestive heart failure (CHF). Durch Proliferationshemmung können nicht nur die bereits eingetretene Krebserkrankung geheilt werden, sondern auch die altersbedingte Entstehungshäufigkeit von Krebs durch NHE-Inhibitoren vermindert und hochsignifikant verzögert werden. Besonders bemerkenswert ist der Befund, dass altersbedingt auftretenden Erkrankungen aller Organe und nicht nur bestimmter Krebsformen unterbunden bzw. hochsignifikant verzögert auftreten. Die Verbindungen der Formel I und/oder deren pharmazeutisch verträgliche Salze eignen sich somit zur Behandlung und insbesondere der Prävention von altersbedingten Formen von Krebs.

Mit NHE-Inhibitoren wird eine zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind: insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen zum Beispiel mit Appetitszüglern erfolgen. Ebenso kann eine Kombination mit blutdrucksenkenden Medikamenten, wie mit ACE-Hemmern, Angiotensinrezeptorantagonisten, Diuretika, Ca⁺²-Antagonisten etc. oder mit stoffwechselnormalisierenden Medikamenten, wie Cholesterinsenkern, gedacht werden.
Die Verbindungen der Formel I und/oder deren pharmazeutisch verträgliche Salze eignen sich somit hervorragend zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung enthaltend eine wirksame Menge einer Verbindung der Formel I und/oder deren pharmazeutisch verträgliche Salze, zusammen mit pharmazeutisch annehmbaren Träger- und Zusatzstoffen, allein oder in Kombination mit anderen pharmakologischen Wirkstoffen oder Arzneimitteln.

Arzneimittel, die eine Verbindung der Formel I und/oder deren pharmazeutisch verträgliche Salze enthalten, können dabei zum Beispiel oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin. Die Arzneimittel enthalten Wirkstoffe der Formel I und/oder deren pharmazeutisch verträgliche Salze im allgemeinen in einer Menge von 0.01 mg bis 1 g pro Dosiseinheit.
Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, intramuskulären oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formel I und/oder deren pharmazeutisch verträgliche Salze in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I und/oder deren pharmazeutisch verträgliche Salze bei einem etwa 75 kg schweren Patienten mindestens 0,001 mg/kg, beispielsweise 0,01 mg/kg, bis höchstens 10 mg/kg, beispielsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, zum Beispiel bis zu 4 Einzeldosen pro Tag. Insbesondere bei i.v. Anwendung, etwa bei einem Infarktpatienten auf der Intensivstation können beispielsweise bis zu 700 mg pro Tag notwendig werden und können die erfindungsgemäßen Verbindungen durch Infusion verabreicht werden.

### Liste der Abkürzungen:

- ADMET: Adsorption - Distribution - Metabolismus -Ausscheidung - Toxikologie
- t-BuOH: 2-Methyl-propan-2-ol
- DCI: desorption chemical ionisation
- DIP: Diisopropylether
- DMF: N,N-Dimethylformamid
- DMSO: Dimethylsulfoxid
- EE: Ethylacetat
- ES: electron spray
- KOtBu: Kalium-2-methyl-propan-2-olat
- MeOH: Methanol
- mp: Schmelzpunkt
- MTB: tert.-Butyl-methylether
- NMP: N-Methyl-2-Pyrrolidon
- RT: Raumtemperatur
- TBTMG: N"-tert-Butyl-N,N,N',N'-tetramethyl-guanidin
- THF: Tetrahydrofuran

### Experimenteller Teil

### Beispiel 1: N-[4-(1,1-Dioxo-isothiazolidin-2-yl)-5-methansulfonyl-2-methyl-benzoyl]-guanidin

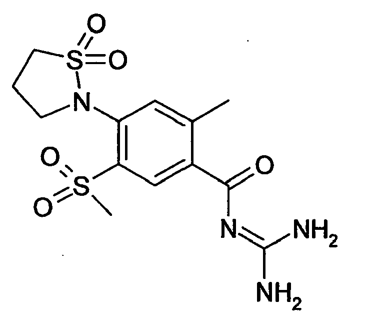

a) 4-(1,1-Dioxo-1-isothiazolidin-2-yl)-5-methansulfonyl-2-methyl-benzoesäuremethylester
   182 mg Isothiazolidine1,1-dioxid (Journal of Organic Chemistry (1987), 52(11), 2162), 369 mg 4-Fluoro-5-methansulfonyl-2-methyl-benzoesäure-methylester (Journal of Medicinal Chemistry (1997), 40(13), 2017) und 1.466 g Cs₂CO₃ wurden in 7.5 ml wasserfreiem DMF 4 Stunden und 30 Minuten gerührt. Das Reaktionsgemisch wurde dann in 120 ml einer halbgesättigten wässrigen NaHCO₃-Lösung gegossen und 3 mal mit je 80 ml EE extrahiert. Über Na₂SO₄ wurde getrocknet und das Solvens im Vakuum entfernt.
   R_{f} (MTB) = 0.44 MS (DCl): 348
b) N-[4-(1,1-Dioxo-isothiazolidin-2-yl)-5-methansulfonyl-2-methyl-benzoyl]-guanidin 726 mg Guanidiniumchlorid wurden in 5 ml wasserfreiem DMF gelöst und bei RT mit einer Lösung von 711 mg KOtBu in 5 ml wasserfreiem DMF versetzt. 10 Minuten wurde bei RT gerührt, dann eine Lösung von 440 mg 4-(1,1-Dioxo-1-isothiazolidin-2-yl)-5-methansulfonyl-2-methyl-benzoesäure-methylester in 5 ml wasserfreiem DMF bei RT zugegeben. Das Gemisch wurde dann 4 Stunden und 45 Minuten bei RTR gerührt, 16 Stunden bei RT stehen gelassen und weitere 90 Minuten bei RT gerührt. Das Reaktionsgemisch wurde dann in 120 ml einer halbgesättigten wässrigen NaHCO₃-Lösung gegossen und 3 mal mit je 80 ml EE extrahiert. Ober Na₂SO₄, wurde getrocknet und das Solvens im Vakuum entfernt und man erhielt 367 mg eines amorphen Feststoffs.
   MS (ES⁺): 374

### Beispiel 2: N-[4-(1, 1 -Dioxo-[1,2]thiazinan-2-yl)-5-methansulfonyl-2-methyl-benzoyl]-guanidin

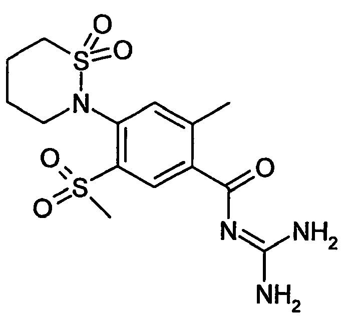

Beispiel 2 wurde analog zu Beispiel 1 synthetisiert.
R_{f} (EE) = 0.50 MS (ES⁺): 388

### Beispiel 3: N-[4-(3,3-Dimethyl-1,1-dioxo-isothiazolidin-2-yl)-5-methansulfonyl-2-methyl-benzoyl]-guanidin

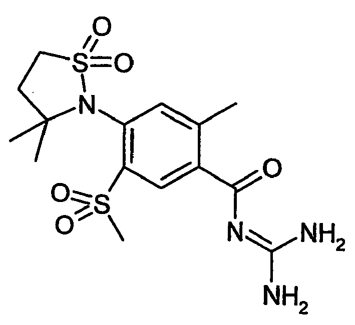

a) 3-Methyl-3-nitro-butan-1-sulfonsäure-phenylester
   Ein Gemisch aus 9.43 g Ethensulfonsäure-phenylester und 4.60 ml 2-Nitropropan wurde auf 80°C erwärmt und bei 80°C 0.89 ml Ethyldiisopropylamin zugetropft. 4 Stunden und 30 Minuten wurde bei 80°C gerührt, 16 Stunden bei RT stehen gelassen, dann weitere 2 Stunden bei 80°C gerührt. Man ließ auf RT abkühlen, dann wurden 250 ml einer 2 N wässrigen HCl-Lösung zugegeben und 3 mal mit je 150 ml EE extrahiert.
   Über Na₂SO₄ wurde getrocknet und das Solvens im Vakuum entfernt, danach wurde 3 mal mit je 100 ml Toluol coevaporiert. Man erhielt 13.47 g eines blassgelben Öls. R_{f} (EE/HEP 1:4) = 0.23
b) 3-Amino-3-methyl-butan-1-sulfonsäure-phenylester
   13.42 g 3-Methyl-3-nitro-butan-l-suffonsäure-phenylester wurden in 40 ml MeOH gelöst und 2.9 g neutral gewaschenes Raney-Nickel zugegeben. 20 Stunden wurde bei RT und 5 bar Wasserstoffdruck hydriert. Dann wurde der Katalysator abfiltriert und das Lösungsmittel im Vakuum entfernt. Man erhielt 11.58 g eines amorphen Feststoffs. R_{f} (EE/MeOH 5:1) = 0.17 MS (DCI): 244
c) 3,3-Dimethyl-isothiazolidine 1,1-dioxid
   11.58 g 3-Amino-3-methyl-butan-1-sulfonsäure-phenylester und 5.34 g KOH wurden in 240 ml THF/95 ml Wasser 7 Stunden unter Rückfluss gekocht. Dann wurden weitere 2.7 g KOH, 50 ml Wasser und 120 ml THF zugegeben und erneut 7 Stunde unter Rückfluss gekocht. Dann wurden weitere 5.34 g KOH zugegeben und erneut 3 Stunden unter Rückfluss gekocht. Man ließ abkühlen, dann wurden 300 ml einer 2 N wässrigen HCl-Lösung zugegeben und 5 mal mit je 250 ml EE extrahiert. Über Na₂SO₄ wurde getrocknet und das Solvens im Vakuum entfernt. Chromatographie an Kieselgel mit EE/HEP 1:1 lieferte 3.82 g weißer Kristalle, mp 72°C.
   R_{f} (EE/HEP 1:2) = 0.14 MS (DCI): 150
d) 4-(3,3-Dimethyl-1,1-dioxo-1-isothiazolidin-2-yl)-5-methansulfonyl-2-methyl-benzoesäure-methylester
   339 mg 4-Fluoro-5-methanesulfonyl-2-methyl-benzoesäure-methylester (Journal of Medicinal Chemistry (1997), 40(13), 2017) und 206 mg 3,3-Dimethyl-isothiazolidine-1,1-dioxid wurden in 7 ml wasserfreiem NMP gelöst und bei RT 0.42 ml N"-tert-Butyl-N,N,N',N'-tetramethyl-guanidin zugegeben.7 Stunden wurde bei 120°C gerührt, dann 16 Stunden stehen gelassen und danach erneut 1 Stunde und 30 Minuten bei 120°C gerührt. Man ließ abkühlen, dann wurde das Reaktionsgemisch mit 120 ml EE verdünnt und 3 mal mit je 80 ml einer 2 N wässrigen HCl-Lösung, dann 3 mal mit je 80 ml einer gesättigten wässrigen Na₂CO₃-Lösung, schließlich noch 1 mal mit 80 ml einer gesättigten wässrigen NaCI-Lösung gewaschen. Über Na₂SO₄ wurde getrocknet und das Solvens im Vakuum entfernt. Chromatographie an Varian Polaris C18-A mit Wasser + 0.1 % Trifluoressigsäure / Acetonitril = 9:1
   Fluss: T = 0 Minuten bis T = 3 Minuten : 50 ml pro Minute
   ab T = 3 Minuten : 150 ml pro Minute
   Man erhielt 50 mg eines farblosen Öls bei einer Retentionszeit von 15.5 Minuten.
   MS (DCI): 376
e) N-[4-(3,3-Dimethyl-1,1-dioxo-isothiazolidin-2-yl)-5-methansulfonyl-2-methyl-benzoyl]-guanidin
   69 mg Guanidiniumchlorid wurden in 2 ml wasserfreiem DMF gelöst und bei RT mit einer Lösung von 67 mg KOtBu in 2 ml wasserfreiem DMF versetzt. 10 Minuten wurde bei RT gerührt, dann eine Lösung von 45 mg 4-(3,3-Dimethyl-1,1-dioxo-1-isothiazolidin-2-yl)-5-methansulfonyl-2-methyl-benzoesäure-methylester in 2 ml wasserfreiem DMF bei RT zugegeben. Das Gemisch wurde dann 3 Stunden bei RT gerührt, 16 Stunden bei RT stehen gelassen und weitere 3 Stundern bei RT gerührt. Das Reaktionsgemisch wurde dann in 80 ml einer halbgesättigten wässrigen NaHCO₃-Lösung gegossen und 3 mal mit je 70 ml MTB extrahiert. Über Na₂SO₄ wurde getrocknet und das Solvens im Vakuum entfernt. Chromatographie an Kieselgel mit EE lieferte 22.5 mg eines viskosen Öls.
   R_{f} (EE/MeOH 5:1) = 0.31 MS (ES⁺): 402
f) N-[4-(3,3-Dimethyl-1,1-dioxo-isothiazolidin-2-yl)-5-methansulfonyl-2-methyl-benzoyl]-guanidin, Hydrochlorid
   22.5 mg N-[4-(3,3-Dimethyl-1,1-dioxo-isothiazolidin-2-yl)-5-methansulfonyl-2-methyl-benzoyl]-guanidin wurden in 5 ml Aceton aufgenommen und mit 0.5 ml einer 4 N wässrigen HCl-Lösung versetzt. Die flüchtigen Bestandteile wurden im Vakuum entfernt und danach 3 mal mit je 5 ml Toluol coevaporiert. Man erhielt 23.9 mg eines amorphen Feststoffs.

### Beispiel 4: N-[4-(1,1-Dioxo-4-phenyl-isothiazolidin-2-yl)-5-methansulfonyl-2-methyl-benzoyl]-guanidin, Hydrochlorid

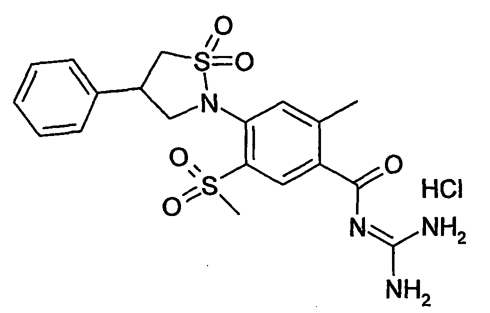

a) 4-(1,1-Dioxo-4-phenyl-isothiazolidin-2-yl)-5-methansulfonyl-2-methyl-benzoesäure-methylester
   600 mg 4-Fluoro-5-methanesulfonyl-2-methyl-benzoesäure-methylester (Journal of Medicinal Chemistry (1997), 40(13), 2017), 600 mg 4-Phenyl-isothiazolidine 1,1-dioxid (Pharmaceutical Chemistry Journal (Translation of Khimiko-Farmatsevticheskü Zhurnal) (2000), Volume Date 1999, 33(11), 598) und 2.379 g Cs₂C0₃ wurden in 20 ml wasserfreiem DMF 4 Stunden bei RT gerührt. Das Reaktionsgemisch wurde dann in 220 ml einer halbgesättigten wässrigen NaHCO₃-Lösung gegossen und 3 mal mit je 140 ml EE extrahiert. Über Na₂SO₄ wurde getrocknet und das Solvens im Vakuum entfernt. Chromatographie an Kieselgel mit DIP lieferte 865 mg eines farblosen Öls.
   R_{f} (DIP) = 0.32 MS (DCI): 424
b) N-[4-(1,1-Dioxo-4-phenyl-isothiazolidin-2-yl)-5-methansulfonyl-2-methyl-benzoyl]-guanidin
   860 mg 4-(1,1-Dioxo-4-phenyl-isothiazolidin-2-yl)-5-methansulfonyl-2-methyl-benzoesäure-methylester wurden analog Beispiel 1b) umgesetzt und man erhielt 737 mg eines farblosen amorphen Feststoffs.
   R_{f} (EE/MeOH 10:1) = 0.54 MS (ES⁺): 450
c) N-[4-(1,1-Dioxo-4-phenyl-isothiazolidin-2-yl)-5-methansulfonyl-2-methyl-benzoyl]-guanidin, Hydrochlorid
   737 mg N-[4-(1,1-Dioxo-4-phenyl-isothiazolidin-2-yl)-5-methansulfonyl-2-methyl-benzoyl]-guanidin wurden in 20 ml Aceton gelöst und 2 ml einer 2N wässrigen HCI-Lösung zugegeben. Anschließend wurden die flüchtigen Bestandteile im Vakuum entfernt. Dann wurde 3 mal mit je 20 ml Toluol coevaporiert und schließlich im Feinvakuum getrocknet. Man erhielt 728 mg weißer Kristalle, mp 259°C (unter Zersetzung).

### Beispiele 5 und 6: N-[4-(1,1-Dioxo-4-phenyl-isothiazolidin-2-yl)-5-methansulfonyl-2-methyl- benzoyl]-guanidin, Hydrochlorid und N-[4-(1,1-Dioxo-4-phenyl-isothiazolidin-2-yl)-5-methansulfonyl-2-methyl-benzoyl]-guanidin, Hydrochlorid

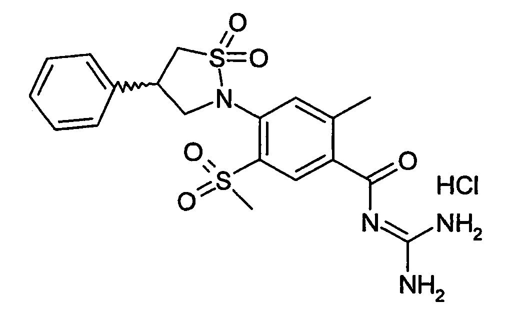

94 mg N-[4-(1,1-Dioxo-4-phenyl-isothiazolidin-2-yl)-5-methansulfonyl-2-methyl-benzoyl]-guanidin, Hydrochlorid wurden chromatographiert an Chiralpack AD/H 32 250x4,6 mit HEP/EtOH/MeOH 1:1:1 und man erhielt die Beispiele 5 und 6:

### Beispiel 5: N-[4-(1,1-Dioxo-4-phenyl-isothiazolidin-2-yl)-5-methansulfonyl-2-methyl-benzoyl]-guanidin, Hydrochlorid, Enantiomer A : 22 mg weißer Kristalle.

Retentionszeit (Chiralpack AD/H 32 250x4,6 mit HEP/EtOH/MeOH 1:1:1): 5.76 Minuten

### Beispiel 6: N-[4-(1,1-Dioxo-4-phenyl-isothiazolidin-2-yl)-5-methansulfonyl-2-methyl-benzoyl]-guanidin, Hydrochlorid, Enantiomer B : 37 mg weißer Kristalle

Retentionszeit (Chiralpack AD/H 32 250x4,6 mit HEP/EtOH/MeOH 1:1:1): 13.05 Minuten

### Beispiel 7: N-{4-[4-(4-Fluor-phenyl)-1,1-dioxo-isothiazolidin-2-yl]-5-methansulfonyl-2-methyl-benzoyl}-guanidin, Hydrochlorid

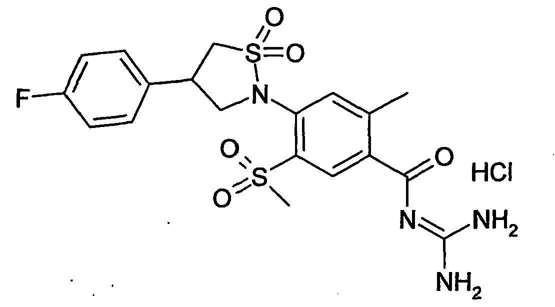

a) 2-(4-Fluor-phenyl)-ethensulfonyl chlorid
   Zu 257 ml auf 3°C gekühltem wasserfreiem DMF wurden 227 ml SO₂Cl₂ zugetropft,
   wobei die Temperatur auf 33°C stieg. 30 Minuten wurde bei RT gerührt, dann wurden 223 ml 1-Fluoro-4-vinyl-benzol zugetropft, wobei die Temperatur der Lösung auf 32°C stieg. Das Reaktionsgemisch wurde langsam auf 60°C Badtemperatur erwärmt, wobei die Innentemperatur 72°C erreichte. 3 Stunden und 45 Minuten wurde bei 70°C gerührt, dann das Reaktionsgemisch portionsweise auf 4 kg Eis gegossen. Dann wurde 1 mal mit 1 l und 5 mal mit je 800 ml MTB extrahiert. Über Na₂SO₄ wurde getrocknet und das Solvens im Vakuum entfernt und man erhielt 163 g eines viskosen Öls, das ohne weitere Reinigung umgesetzt wurde.
   R_{f} (EE/HEP 1:4) = 0.49
b) 2-(4-Fluoro-phenyl)-ethensulfonsäure-phenylester
   163 g 2-(4-Fluor-phenyl)-ethensulfonyl chlorid wurden in 1.5 I Toluol gelöst und bei RT 69.64 g Phenol zugegeben. Anschließend wurden bei RT 103 ml Triethylamin zugetropft, dabei stieg die Temperatur auf 44°C. Dann wurde das Gemisch 90 Minuten bei RT gerührt, 16 Stunden stehen gelassen und weitere 2 Stunden bei RT gerührt. Dann wurde das Lösungsmittel im Vakuum entfernt und das Rohprodukt mit 2.5 I EE aufgenommen. 3 mal wurde mit 750 ml einer gesättigten wäßrigen NaHCO3-Lösung gewaschen, anschließend wurde 3 mal mit je 750 ml einer 2 N wäßrigen HCl-Lösung gewaschen. Über Na₂SO₄ wurde getrocknet und das Solvens im Vakuum entfernt.
   Das Produkt wurde anschließend in 350 ml HEP 1 Stunde bei 70°C gerührt, beim Abkühlen kristallisierte das Produkt aus und wurde mit HEP gewaschen und im Feinvakuum getrocknet. Man erhielt 145.0 g weißer Kristalle, mp 108°C.
   R_{f} (EE/HEP 1:6) = 0.26 MS (DCI): 279
c) 2-(4-Fluor-phenyl)-2-nitro-ethansulfonsäure-phenylester
   56.3 g Natriummethanolat wurden in 1 l DMSO aufgenommen und bei RT 56.3 ml Nitromethan zugetropft. Dabei stiegt die Innentemperatur auf 40°C. Dann wurde bei einer Temperatur zwischen 26°C und 30°C eine Lösung von 145.0 g 2-(4-Fluorophenyl)-ethensulfonsäure-phenylester in 1 I DMSO zugetropft. 1 Stunde wurde bei RT gerührt, dann 16 Stunden bei RT stehen gelassen, dann weitere 6 Stunden und 30 Minuten bei RT gerührt. Das Reaktionsgemisch wurde dann auf 8 kg Eis gegossen und 5 mal mit je 2 I EE extrahiert. Über Na₂SO₄ wurde getrocknet und das Solvens im Vakuum entfernt. Chromatographie an Kieselgel mit EE/HEP 1:5 lieferte 77.35 g eines farblosen Öls.
   R_{f} (EE/HEP 1:5) = 0.19
d) 3-Amino-2-phenyl-propan-1-sulfonsäure phenylester
   71.00 g 2-(4-Fluor-phenyl)-2-nitro-ethansulfonsäure-phenylester wurden in 200 ml MeOH und 50 ml THF gelöst und 2 g neutral gewaschenes Raney-Nickel zugegeben. 40 Stunden wurde bei RT und 5.5 bar Wasserstoffdruck hydriert. Dann wurde der Katalysator abfiltriert und das Lösungsmittel im Vakuum entfernt. Man erhielt 61.01 g eines amorphen Feststoffs.
   R_{f} (EE/MeOH 5:1) = 0.38 MS (ES⁺): 309
e) 4-(4-Fluor-phenyl)-isothiazolidin 1,1-dioxid
   61.01 g 3-Amino-2-phenyl-propan-1-sulfonsäure phenylester wurden in 800 ml THF gelöst und 80 ml Wasser sowie 22.23 g KOH zugegeben. 15 Stunden wurde bei RT gerührt, dann wurde 5 Stunden unter Rückfluss gekocht. Anschließend wurden das Lösungsmittel im Vakuum entfernt, der Rückstand mit 1 l einer gesättigten wässrigen NaHSO₄-Lösung aufgenommen und 3 mal mit je 500 ml EE extrahiert. Über MgSO₄ wurde getrocknet und das Solvens im Vakuum entfernt. Chromatographie an Kieselgel mit DIP lieferte 29.00 g farbloser Kristalle, mp 155°C.
   R_{f} (DIP) = 0.18 MS (DCl): 215
f) 4-[4-(4-Fluor-phenyl)-1,1-dioxo-isothiazolidin-2-yl]-5-methansulfonyl-2-methyl-benzoesäure-methylester
   18.00 g 4-(4-Fluor-phenyl)-isothiazolidin 1,1-dioxid, 20.59 g 4-Fluoro-5-methansulfonyl 2-methyl-benzoesäure-methylester (Journal of Medicinal Chemistry (1997), 40(13), 2017) und 81.75 g Cs₂CO₃ wurden in 500 ml wasserfreiem DMF 5 Stunden bei RT gerührt. Dann wurden weitere 2.00 g 4-Fluoro-5-methansulfonyl-2-methyl-benzoesäure-methylester zugegeben, 1 Stunde bei RT gerührt und 15 Stunden bei RT stehen gelassen. Das Reaktionsgemisch wurde auf 1800 ml Wasser gegossen, 1 Stunde bei RT gerührt und das Produkt abgesaugt. Das Produkt wurde dann in 1 l EE gelöst, die Lösung mit MgSO₄ und Aktivkohle versetzt und 15 Minuten bei RT gerührt.
   Dann wurde abfiltriert und das Lösungsmittel im Vakuum entfernt. Man erhielt 35.00 g eines farblosen Schaumes.
   R_{f} (DIP) = 0.21 MS (ES-): 441
g) N-{4-[4-(4-Fluor-phenyl)-1,1-dioxo-isothiazolidin-2-yl]-5-methansulfonyl-2-methyl-benzoyl}-guanidin
   44.48 g KOtBu wurden in 800 ml wasserfreiem DMF gelöst und bei RT 45.44 g Guanidiniumchlorid zugegeben. 1 Stunde wurde bei RT gerührt und dann die Lösung zu einer Lösung von 35.00 g 4-[4-(4-Fluor-phenyl)-1,1-dioxo-isothiazolidin-2-yl]-5-methansulfonyl-2-methyl- benzoesäure-methylester in 300 ml wasserfreiem DMF gegossen. Das Gemisch wurde dann 5 Stunden bei RT gerührt. Dann wurde auf 2 l Wasser gegossen, mit wässriger HCl-Lösung auf pH=8 gestellt und 5 mal mit je 300 ml EE extrahiert. Über MgSO₄ wurde getrocknet und das Solvens im Vakuum entfernt.
   Aus EE wurde umkristallisiert und man erhielt 27.50 g farbloser Kristalle, mp 177-178°C.
   R_{f} (EE/MeOH 10: 1) = 0.45 MS (ES⁺): 468
h) N-{4-[4-(4-Fluor-phenyl)-1,1-dioxo-isothiazolidin-2-y)]-5-methansulfonyl-2-methyl-benzoyl}-guanidin, Hydrochlorid
   562 mg N-{4-[4-(4-Fluor-phenyl)-1,1-dioxo-isothiazolidin- 2-yl]-5-methansulfonyl-2-methyl-benzoyl}-guanidin wurden in 20 ml Aceton gelöst und 2 ml einer 4 N wässrigen HCl-Lösung versetzt. Die flüchtigen Bestandteile wurden im Vakuum entfernt, dann wurde 3 mal mit je 20 ml Toluol coevaporiert und schließlich im Feinvakuum getrocknet. Man erhielt 587 mg weißer Kristalle, mp 250°C (unter Zersetzung).

### Beispiele 8 und 9: N-{4-[4-(4-Fluor-phenyl)-1,1-dioxo-isothiazolidin-2-yl]-5-methansulfonyl-2- methyl-benzoyl}-guanidin, Hydrochlorid und N-{4-[4-(4-Fluorphenyl)-1,1-dioxo-isothiazolidin-2-yl]-5-methansulfonyl-2- methyl-benzoyl}-guanidin, Hydrochlorid

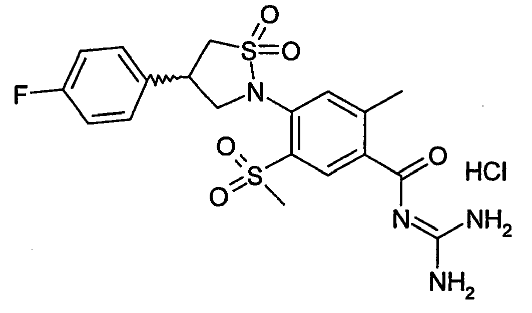

Zur Enantiomerentrennung der Titelverbindung des Beispiel 7 wurden 27.50 g des Racemats 7g) an einer mit Chiralpack AD/10, 20µM gefüllten Säule der Abmessungen 400mm x 100mm chromatographiert mit HEP/EtOH/MeOH 2:1:1, 300 ml pro Minute, und weiter umgesetzt zu den Beispiele 8 und 9:

### Beispiel 8: N-{4-[4-(4-Fluor-phenyl)-1,1-dioxo-isothiazolidin-2-yl]-5-methansulfonyl-2-methyl-benzoyl}-guanidin, Hydrochlorid, Enantiomer A

a) N-{4-[4-(4-Fluor-phenyl)-1,1-dioxo-isothiazolidin-2-yl]-5-methansulfonyl-2-methyl-benzoyl}-guanidin Enantiomer A, Retentionszeit oben genanntes System: 16.5 Minuten
   12.2 g weißer Kristalle; [□]^{25°C}D = -49.3
b) N-{4-[4-(4-Fluor-phenyl)-1,1-dioxo-isothiazolidin-2-yl]-5-methansulfonyl-2-methyl-benzoyl}-guanidin, Hydrochlorid, Enantiomer A
   84 mg N-{4-[4-(4-Fluor-phenyl)-1,1-dioxo-isothiazolidin-2-yl]-5-methansulfonyl-2-methyl-benzoyl}-guanidin Enantiomer A wurden analog Beispiel 7h) in das Hydrochlorid überführt und man erhielt 90 mg weißer Kristalle.

### Beispiel 9: N-{4-[4-(4-Fluor-phenyl)-1,1-dioxo-isothiazolidin-2-yl]-5-methansulfonyl-2-methyl-benzoyl}-guanidin, Hydrochlorid, Enantiomer B

a) N-{4-[4-(4-Fluor-phenyl)-1,1-dioxo-isothiazolidin-2-yl]-5-methansulfonyl-2-methyl-benzoyl}-guanidin, Enantiomer B, Retentionszeit oben genanntes System: 22.0 Minuten
   11.8 g weißer Kristalle; [□]^{25°C}D = +49.4
b) N-{4-[4-(4-Fluor-phenyl)-1,1-dioxo-isothiazolidin-2-yl]-5-methansulfonyl-2-methyl-benzoyl}-guanidin, Hydrochlorid, Enantiomer B
   5.00 g N-{4-[4-(4-Fluor-phenyl)-1,1-dioxo-isothiazolidin-2-yl]-5-methansulfonyl-2-methyl-benzoyl}-guanidin Enantiomer B wurden analog Beispiel 7h) in das Hydrochlorid überführt und man erhielt 5.32 g weißer Kristalle.

### Beispiel 10: N-[4-(1,1-Dioxo-4-phenyl-1-[1,2]thiazinan-2-yl)-5-methansulfonyl-2-methyl-benzoyl]-guanidin

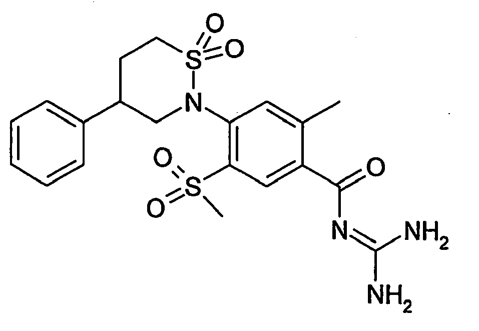

Beispiel 10 wurde analog zu Beispiel 4 synthetisiert unter Verwendung von 4-Phenyl-[1,2]thiazinane 1,1-dioxid (J. Org. Chem. 1991, 56, 3549) als Startmaterial.
R_{f} (EE/MeOH 5:1) = 0.40 MS (ES⁺): 464

### Beispiel 11: N-[4-(5-Cyclopropyl-1,1-dioxo-1-[1,2,5]thiadiazolidin-2-yl)-5-methansulfonyl-2- methyl-benzoyl]-guanidin

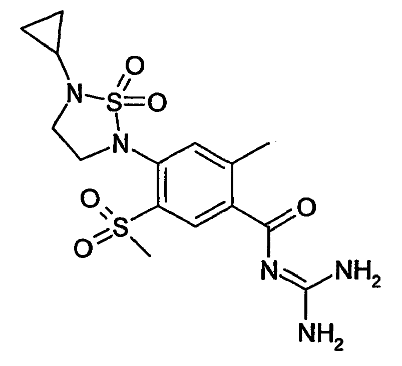

a) Cyclopropylsulfamoyl-carbaminsäure-tert-butylester 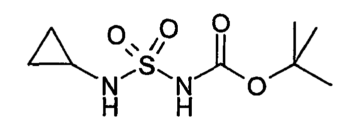 1.33 ml t-BuOH wurden in 50 ml wasserfreiem CH₂Cl₂ gelöst und bei RT 2.00 g Chlorsulfonylisocyanat zugetropft. Dabei steigt die Innentemperatur auf 30°C. 1 Stunde wurde bei RT gerührt, dann wurden 1.96 ml Cyclopropylamin zugetropft und das Reaktionsgemisch 16 Stunden bei RT stehen gelassen. Dann wurde 3 mal mit je 15 ml Wasser gewaschen. Über Na₂SO₄ wurde getrocknet und das Solvens im Vakuum entfernt. Man erhielt 1.3 g eines amorphen Feststoffs.
   MS (DCI): 237
b) 5-Cyclopropyl-1,1-dioxo-[1,2,5]thiadiazolidin-2-carbonsäure-tert-butylester 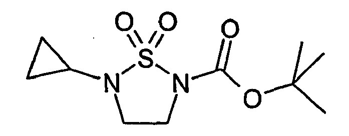 410 mg Cyclopropylsulfamoyl-carbaminsäure-tert-butylester, 150 µl 1,2-Dibromethan und 719 mg K₂CO₃ wurden in 6 ml wasserfreiem Aceton 9 Stunden unter Rückfluss gekocht. Das Reaktionsgemisch wurde 15 Stunden bei RT stehen gelassen. Dann wurden 20 mg Tetrabutylammoniumiodid zugegeben und 9 Stunden unter Rückfluss gekocht. Dann wurde mit 100 ml EE verdünnt und 2 mal mit je 10 ml Wasser gewaschen. Über Na₂SO₄ wurde getrocknet und das Solvens im Vakuum entfernt.
   Man erhielt 400 mg eines farblosen Öls.
   MS (DCI): 263
c) 2-Cycfopropyl-[1,2,5]thiadiazolidin 1,1-dioxid 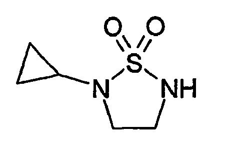 400 mg 5-Cyclopropyl-1,1-dioxo-[1,2,5]thiadiazolidin-2-carbonsäure-tert-butylester wurden in 10 ml CH₂Cl₂/Trifluoressigsäure 1:1 gelöst und 2 Stunden stehen gelassen. Die flüchtigen Bestandteile wurden im Vakuum entfernt und 2 mal mit je 50 ml CH₂Cl₂ coevaporiert. Man erhielt 410.mg eines blassgelben Öls, das direkt weiter eingesetzt wurde.
d) 4-(5-Cyclopropyl-1,1-dioxo-[1,2,5]thiadiazolidin-2-yl)-5-methansulfonyl-2-methyl-benzoesäure-methylester 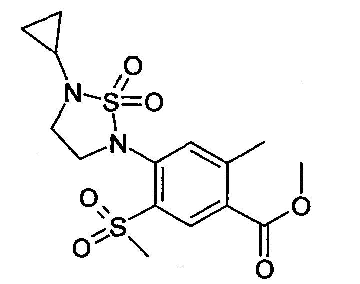 203 mg 2-Cyclopropyl-[1,2,5]thiadiazolidin 1,1-dioxid, 308 mg 4-Fluoro-5-methansulfonyl-2-methyl-benzoesäure-methylester und 2.44 g Cs₂CO₃ wurden in 25 ml wasserfreiem DMF 5 Stunden bei 80°C gerührt. 2 Tage wurde bei RT stehen gelassen, dann mit 200 ml EE verdünnt und 3 mal mit je 20 ml Wasser gewaschen. Über Na₂SO₄ wurde getrocknet und das Solvens im Vakuum entfernt. Chromatographie an Kieselgel mit DIP lieferte 230 mg eines amorphen Feststoffs.
   R_{f} (DIP) = 0.13 MS (DCl): 389
e) N-[4-(5-Cyclopropyl-1,1-dioxo-1-[1,2,5]thiadiazolidin-2-yl)-5-methansulfonyl-2-methyl-benzoyl]-guanidin
   191 mg KOtBu wurden in 10 ml wasserfreiem DMF gelöst und bei RT 260 mg Guanidiniumchlorid zugegeben. 30 Minuten wurde bei RT gerührt und dann die Lösung zu 220 mg 4-(5-Cyclopropyl-1,1-dioxo-[1,2,5]thiadiazolidin-2-yl)-5= methansulfonyl-2-methyl- benzoesäure-methylester gegossen. Das Gemisch wurde dann 5 Stunden bei RT gerührt. Dann wurde auf 50 ml Wasser gegossen, mit wässriger HCI-Lösung auf pH=8 gestellt und 3 mal mit je 30 ml EE extrahiert. Über Na₂S0₄ wurde getrocknet und das Solvens im Vakuum entfernt. Chromatographie an Kieselgel mit EE/MeOH 10:1 lieferte 163 mg eines amorphen Feststoffs.
   R_{f} (EE/MeOH 10:1) = 0.28 MS (ES⁺): 415

### Beispiel 12: N-[4-(6-Cyclopropyl-1,1-dioxo-1-[1,2,6]thiadiazinan-2-yl)-5-methansulfonyl-2- methyl-benzoyl]-guanidin

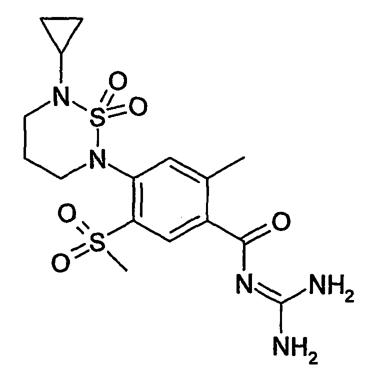

Beispiel 12 wurde analog zu Beispiel 11 synthetisiert.
R_{f} (EE/MeOH 10:1) = 0.29 MS (ES⁺): 429

### Beispiel 13: N-[4-(1,1-Dioxo-1-isothiazolidin-2-yl)-3-trifluoromethyl-benzoyl]-guanidin

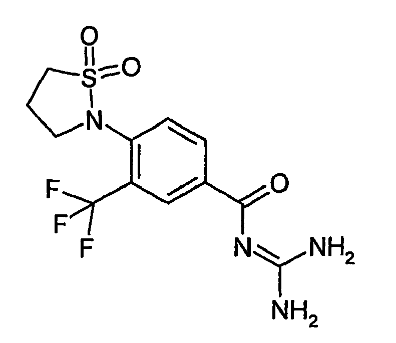

Beispiel 13 wurde analog zu Beispiel 1 synthetisiert unter Verwendung von 4-Fluor-3-trifluormethyl-benzoesäuremethylester als Startmaterial.
Rf (EE/MeOH 10:1) = 0.53 MS (ES⁺) 351
a) 4-Fluor-3-trifluormethyl-benzoesäuremethylester
   5 g 4-Fluor-3-trifluormethyl-benzoesäure und 9 ml SOCl₂ wurden in 50 ml MeOH 8 h bei 60□C gerührt. Anschließend wurden die flüchtigen Bestandteile im Vakuum entfernt und man erhielt 5,1 g eines farblosen Öls, das ohne Reinigung weiter eingesetzt wurde.
   Rf (EE/MeOH 10:1) = 0.74 MS (DCI) 223

### Beispiel 14: N-[4-(1,1-Dioxo-1-[1,2]thiazinan-2-yl)-3-trifluoromethyl-benzoyl]-guanidin

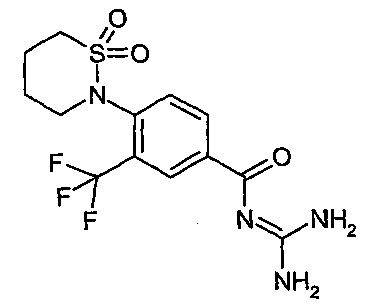

Beispiel 14 wurde analog zu Beispiel 1 synthetisiert unter Verwendung von 4-Fluor-3-trifluormethyl-benzoesäuremethylester als Startmaterial (Synthese von 4-Fluor-3-trifluormethyl-benzoesäuremethylester wie unter Beispiel 13 a))
Rf (EE/MeOH 10:1) = 0.62

### Bestimmung der NHE-Hemmung

Die Hemmkonzentration IC₅₀ für die NHE-1 Hemmung wurde wie folgt bestimmt:

IC₅₀ für die NHE-1 Hemmnung wurden bestimmt in einem FLIPR-Assay mittels Messung der pHᵢ-Erholung in transfizierten Zelllinien, die den humanen NHE-1 exprimieren.
Der Assay wurde im FLIPR (Fluorometric imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließender Selektion keine endogene NHE-Aktivität auf), wurden am Vortag mit einer Dichte von ∼25.000 Zellen / Well ausgesät. Das Wachstumsmedium der transfizierten Zellen (Iscove +10 % fötales Kälberserum) enthielt zusätzlich G418 als Selektionsantibiotikum, um die Anwesenheit der transfizierten Sequenzen sicherzustellen.

Der eigentliche Assay begann mit der Entfernung des Wachstumsmediums und Zugabe von 100 µl/Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchlorid, 1 mM MgCl₂, 1 mM CaCl₂, 5 mM KCl, 20 mM HEPES, 5 mM Glucose; pH 7,4 [mit KOH eingestellt]). Die Zellen wurden dann 20 Minuten bei 37°C inkubiert. Diese Inkubation führte zur Beladung der Zellen mit dem fluoreszierenden Farbstoff, dessen Fluoreszenzintensität vom pHi abhängt, und mit NH₄Cl, was zu einer leichten Alkalinisierung der Zellen führte.
Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.

Nach dieser 20-minütigen Inkubation wurde der Beladungspuffer, der NH₄Cl und freies BCECF-AM enthielt, durch dreimaliges Waschen im Zellwasher (Tecan Columbus) mit jeweils 400 µl Waschpuffer (133,8 mM Cholinchlorid, 4,7 mM KCl, 1,25 mM MgCl₂, 1,25 mM CaCl₂, 0,97 mM K₂HPO₄, 0,23 mM KH₂PO₄, 5 mM HEPES, 5 mM Glucose pH 7,4 [mit KOH eingestellt]) entfernt. Das in den Wells verbleibende Restvolumen betrug 90 µl (50 -125 µl möglich). Dieser Waschschritt entfernte das freie BCECF-AM und führte als Folge der Entfernung der externen NH₄⁺-Ionen zu einer intrazellulären Ansäuerung (~ pHi 6.3 - 6.4).

Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wurde, führte der Waschprozess dazu, dass intrazellulär H⁺ zurückblieb, was die Ursache für die intrazelluläre Ansäuerung war. Diese kann letztlich zum Zelltod führen, wenn sie lang genug anhält. An dieser Stelle war es wichtig, dass der Waschpuffer natriumfrei (<1 mM) war, da extrazelluläre Natrium-Ionen zu einer augenblicklichen Erholung des pHᵢ durch die Aktivität der klonierten NHE-Isoformen führen würde.

Es war ebenfalls wichtig, dass alle verwendeten Puffer (Beladungspuffer, Waschpuffer, Recoverypuffer) keine HCO₃⁻-Ionen enthielten, da die Anwesenheit von Bicarbonat zur Aktivierung störender bicarbonatabhängiger pHᵢ-Regulationssysteme führen würde, die in der parentalen LAP-1 Zelllinie enthalten sind.

Die Mikrotiterplatten mit den angesäuerten Zellen wurden dann (bis zu 20 Minuten nach der Ansäuerung) zum FLIPR transferiert. Im FLIPR wurde der intrazelluläre Fluoreszenzfarbstoff durch Licht mit einer Wellenlänge von 488 nm, das von einem Argon-Laser erzeugt wurde, angeregt, und die Messparameter (Laserleistung, Belichtungszeit und Blende der im FLIPR eingebauten CCD-Kamera) wurden so gewählt, dass das durchschnittliche Fluoreszenzsignal pro Well zwischen 30000 und 35000 relativen Fluoreszenzeinheiten lag.

Die eigentliche Messung im FLIPR begann damit, dass Software-gesteuert alle zwei Sekunden eine Aufnahme mit der CCD-Kamera gemacht wurde. Nach zehn Sekunden wurde die Erholung des intrazellulären pH's durch Zugabe von 90 µl Recoverypuffer (133,8 mM NaCl, 4,7 mM KCl, 1,25 mM MgCl₂, 1,25 mM CaCl₂, 0,97 mM K₂HPO₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.

Als Positivkontrollen (100 % NHE-Aktivität) dienten Wells, denen reiner Recoverypuffer zugegeben wurde, Negativkontrollen (0 % NHE-Aktivität) erhielten Waschpuffer. In allen anderen Wells wurde Recoverypuffer mit der zweifach konzentrierten Testsubstanz hinzugegeben. Die Messung im FLIPR endete nach 60 Messpunkten (zwei Minuten).

Die Rohdaten werden in das Programm ActivityBase exportiert. Mit diesem Programm werden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear war, sondern am Ende aufgrund abnehmender NHE-Aktivität bei höheren pHᵢ-Werten abfiel, war es wichtig, für die Auswertung der Messung den Teil auszuwählen, in dem die Fluoreszenzzunahme der Positivkontrollen linear war.

| Beispiel | NHE1-Hemmung IC₅₀ [nM] |
|---|---|
| 1 | 38 |
| 2 | 144 |
| 3 | 195 |
| 4 | 6 |
| 5 | 13 |
| 6 | 45 |
| 7 | 19 |
| 8 | 12 |
| 9 | 23 |
| 10 | 51 |
| 11 | 113 |
| 12 | 1522 |
| 13 | 20 |
| 14 | 13 |

### In vivo Pharmakokinetik - Profilierung mit der "n in one Methode"

Als pharmakokinetische Kenndaten wurden die Expositionsdaten und die Halbwertszeit wie folgt bestimmt:

Der erfindungsgemäße NHE-1 Inhibitor des Beispiels 9 und als Referenzsubstanz der bekannte NHE-1 Hemmer Cariporid mit der Formel 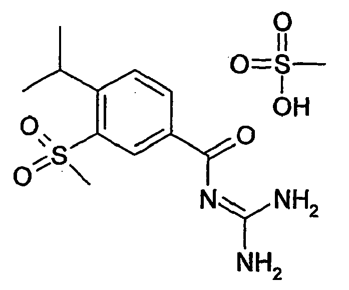 wurden in wässrigem, leicht saurem Medium (Wasser, pH 4, eingestellt mit 1 M Salzsäure) gelöst. Die Konzentration der so hergestellten wässrigen Formulierung betrug ca. 1,5 mg je Substanz pro 1 g Lösung. 10 ml dieser Formulierung wurden einem männlichen, nüchternen Beagle-Hund in die Vena jugularis mittels Katheter als Bolus einmal appliziert (Dosis ca. 1 mg je applizierter Substanz pro kg Körpergewicht des Hundes). Mittels eines zweiten Katheters wurden nach 5min, 15min, 30min, 1 h, 2h, 4h, 8h und 24 h Blutproben gewonnen und heparinisiertes Plasma durch Zentrifugation bei 1000G in entsprechenden Plasmaröhrchen hergestellt.

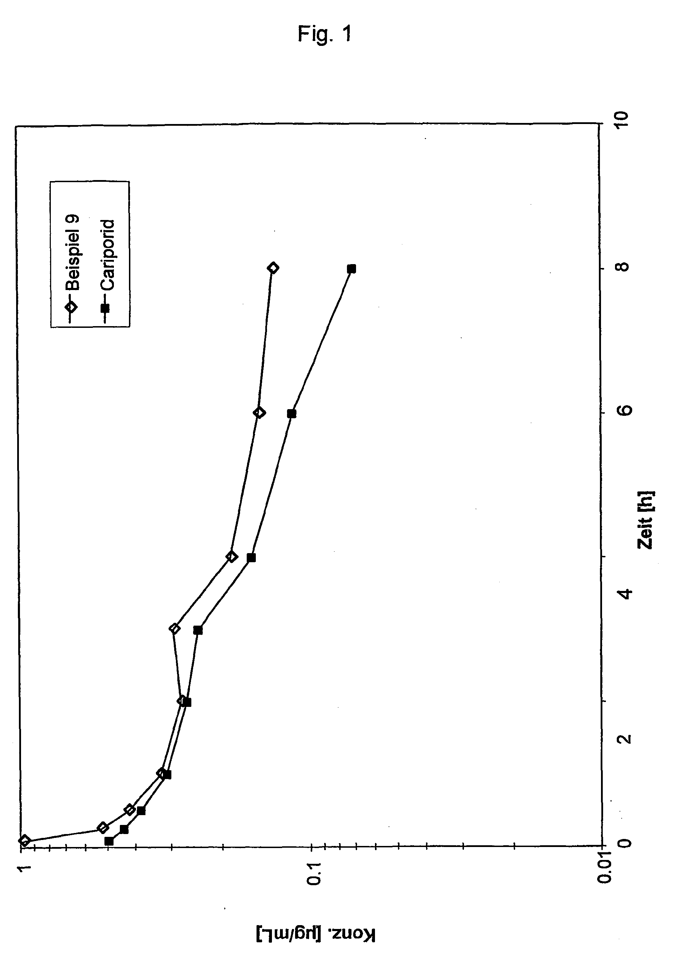

Die Plasmaproben wurden aufgearbeitet und nach einer HPLC-Trennung mittels MS/MS quantifiziert. Diese Methode erlaubte wegen ihrer hohen Spezifität die gleichzeitige Bestimmung mehrerer Substanzen. Aus den Konzentrations - Zeitkurven (siehe Abbildung 1) ließen sich die Expositionen mittels des Computerprogramms WinNonlin berechnen und mit der Exposition der bekannten NHE-1 Referenzsubstanz vergleichen. Da die verschiedenen Substanzen im gleichen Tier zum gleichen Zeitpunkt gemessen wurden, ergab sich ein exakter Vergleich der Verbindungen und es konnte ein Ranking der Verteilungsvolumina erstellt werden.

| Verbindung | Halbwertszeit [h] |
|---|---|
| Beispiel 9 | 4.6 |
| Referenzsubstanz Cariporid | 3.3 |

Aus den Konzentrations-Zeitkurven in Abbildung 1 ist deutlich ersichtlich, das die erfindungsgemäße Verbindung im Blut eine deutlich höhere Exposition zeigt als die Referenzsubstanz Cariporid.

In der Abbildung wurden folgende Beschriftungen und Kennzeichnungen vorgenommen:
Fig. 1: Konzentrations - Zeitkurven im Blutplasma von Hunden nach Gabe von je ca. 1 mg/kg von der Verbindung des Beispiels 9 und von Cariporid.
Y-Achse: Konzentration der gemessenen Verbindung in µg/ml im Plasma
X-Achse: Zeit in h

## Patentansprüche

1. Verbindungen der Formel I 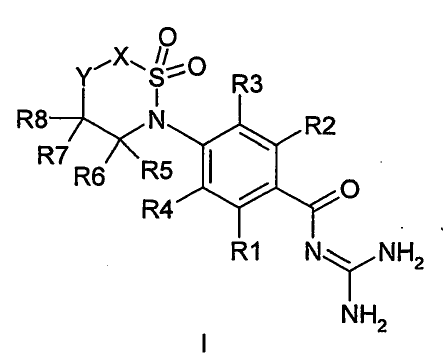 worin bedeuten
R1 Wasserstoff, Alkyl mit 1, 2, 3 oder 4 C-Atomen, Alkoxy mit 1, 2, 3 oder 4 C-Atomen, F, Cl, Br, I, -CN, NR13R14, -O-(CH₂)ₙ-(CF₂)ₒ-CF₃ oder -(SOₘ)_{q}-(CH₂)ᵣ-(CF₂)ₛ-CF₃;
R13 und R14 unabhängig voneinander Wasserstoff, Alkyl mit 1, 2, 3 oder 4 C-Atomen oder -CH₂-CF₃;
m Null, 1 oder 2
n, o, q, r und s unabhängig voneinander Null oder 1;
R2 Wasserstoff, Alkyl mit 1, 2, 3 oder 4 C-Atomen, Alkoxy mit 1, 2, 3 oder 4 C-Atomen, F, Cl, Br, I, -CN, NR15R16, -O-(CH₂)ᵤ-(CF₂)ᵥ-CF₃ oder -(SO_{w})ₓ-(CH₂)_{y}-(CF₂)_{z}-CF₃;
R15 und R16 unabhängig voneinander Wasserstoff, Alkyl mit 1, 2, 3 oder 4 C-Atomen oder -CH₂-CF₃;
w Null, 1 oder 2
u, v, x, y und z unabhängig voneinander Null oder 1;
R3 Wasserstoff, F, Cl, Br, I, -CN, -SO₂CH₃, Alkoxy mit 1, 2, 3 oder 4 C-Atomen, NR9R10, -O-(CH₂)_{b}-(CF₂)_{c}-CF₃, -(SO_{d})ₑ-(CH₂)_{f}-(CF₂)_{g}-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R9 und R10 unabhängig voneinander Wasserstoff, Alkyl mit 1, 2, 3 oder 4 C-Atomen oder -CH₂-CF₃;
b,c,e und g unabhängig voneinander Null oder 1;
d Null, 1 oder 2;
f Null, 1, 2, 3 oder 4;
oder
R3 -(CH₂)ₕ -Phenyl oder -O-Phenyl,
in denen die Phenylreste unsubstituiert sind oder substituiert sind mit 1, 2 oder 3 Resten ausgewählt aus der Gruppe bestehend aus Reihe F, Cl, Br, I, -Oⱼ-(CH₂)ₖ-CF₃, Alkoxy mit 1, 2, 3 oder 4 C-Atomen, Alkyl mit 1, 2, 3 oder 4 C-Atomen und -SO₂CH₃;
j Null oder 1;
k Null, 1, 2 oder 3;
h Null, 1, 2, 3 oder 4;
oder
R3 -(CH₂)ₐₐ -Heteroaryl,
das unsubstituiert ist oder substituiert ist mit 1, 2 oder 3 Resten ausgewählt aus der Gruppe bestehend aus F, Cl, Br, I, -O_{bb}-(CH₂)_{cc}-CF₃, Alkoxy mit 1, 2, 3 oder 4 C-Atomen, Alkyl mit 1, 2, 3 oder 4 C-Atomen und -SO₂CH₃;
bb Null oder 1;
cc Null, 1, 2 oder 3;
aa Null, 1, 2, 3 oder 4;
R4 Wasserstoff, F, Cl, Br, I, -CN, -SO₂CH₃ Alkoxy mit 1, 2, 3 oder 4 C-Atomen, NR11R12, -O-(CH₂)ₑₑ-(CF₂)_{ff}-CF₃, -(SO_{gg})ₕₕ-(CH₂)ⱼⱼ-(CF₂)ₖₖ-CF₃, Alkyl mit 1, 2, 3, 4, 5 oder 6 C-Atomen oder Cycloalkyl mit 3, 4, 5, 6, 7 oder 8 C-Atomen, in dem 1, 2, 3 oder 4 Wasserstoff-Atome durch Fluoratome ersetzt sein können;
R11 und R12 unabhängig voneinander Wasserstoff, Alkyl mit 1, 2, 3 oder 4 C-Atomen oder -CH₂-CF₃;
ee, ff, hh und kk unabhängig voneinander Null oder 1;
gg Null, 1 oder 2;
jj Null, 1, 2, 3 oder 4;
oder
R4 -(CH₂)ₗₗ -Phenyl oder -O-Phenyl,
in denen die Phenylreste unsubstituiert sind oder substituiert sind mit 1, 2 oder 3 Resten ausgewählt aus der Gruppe bestehend aus Reihe F, Cl, Br, I, -Oₘₘ-(CH₂)ₙₙ-CF₃, Alkoxy mit 1, 2, 3 oder 4 C-Atomen, Alkyl mit 1, 2, 3 oder 4 C-Atomen und -SO₂CH₃;
mm Null oder 1;
nn Null, 1, 2 oder 3;
II Null, 1, 2, 3 oder 4;
oder
R4 -(CH₂)ₒₒ -Heteroaryl,
das unsubstituiert ist oder substituiert ist mit 1, 2 oder 3 Resten ausgewählt aus der Gruppe bestehend aus F, Cl, Br, I, -Oₚₚ-(CH₂)ᵣᵣCF₃, Alkoxy mit 1, 2, 3 oder 4 C-Atomen, Alkyl mit 1, 2, 3 oder 4 C-Atomen und -SO₂CH₃;
pp Null oder 1;
rr Null, 1, 2 oder 3;
oo Null, 1, 2, 3 oder 4;
R5 und R6 unabhängig voneinander Wasserstoff oder Alkyl mit 1, 2, 3 oder 4 C-Atomen;
R7 und R8 unabhängig voneinander Wasserstoff, Alkyl mit 1, 2, 3 oder 4 C-Atomen, Cycloalkyl mit 3, 4, 5, 6, 7 oder 8 C-Atomen, -CF₃ oder Phenyl, wobei der Phenylrest unsubstituiert ist oder substituiert ist mit 1, 2 oder 3 Resten ausgewählt aus der Gruppe bestehend aus Reihe F, Cl, Br, I, -Oₛₛ-(CH₂)ₜₜ-CF₃, Alkoxy mit 1, 2, 3 oder 4 C-Atomen, Alkyl mit 1, 2, 3 oder 4 C-Atomen und -SO₂CH₃;
ss Null oder 1
tt Null, 1, 2 oder 3;
oder
R7 und R8 bilden zusammen mit dem sie tragenden C-Atom ein Cycloalkyl mit 3, 4, 5, 6, 7 oder 8 C-Atomen;
X -CH₂- oder -NR17-
R17 Wasserstoff, Alkyl mit 1, 2, 3 oder 4 C-Atomen, Cycloalkyl mit 3, 4, 5, 6, 7 oder 8 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, I, -Oᵤᵤ-(CH₂)ᵥᵥ-CF₃, Alkoxy mit 1, 2, 3 oder 4 C-Atomen, Alkyl mit 1, 2, 3 oder 4 C-Atomen und -SO₂CH₃;
uu Null oder 1;
w Null, 1, 2 oder 3;
Y eine Bindung oder eine Alkylenkette mit 1, 2 oder 3 C-Atomen;
sowie deren pharmazeutisch verträgliche Salze.

2. Verbindungen der Formel I nach Anspruch 1, in denen bedeuten:
R1 Wasserstoff, Alkyl mit 1, 2, 3 oder 4 C-Atomen, Alkoxy mit 1, 2, 3 oder 4 C Atomen, F, Cl, NR13R14, -O-(CH₂)ₙ-(CF₂)ₒ-CF₃ oder -(SOₘ)_{q}-(CH₂)ᵣ-(CF₂)ₛ-CF₃;
R13 und R14 unabhängig voneinander Wasserstoff, Methyl, Ethyl oder -CH₂-CF3;
m Null, 1 oder 2
n, o, q, r und s unabhängig voneinander Null oder 1;
R2 Wasserstoff, Methyl, Methoxy, F, Cl, -O-CF₃, -O-CH₂-CF₃ oder -S-CF₃;
R3 Wasserstoff, F, Cl, -CN, -SO₂CH₃, Methoxy, Ethoxy, NR9R10, -O-CF₃, -O-CH₂-CF₃, -SO₂CF₃, -S-CF₃, -CF₃, Alkyl mit 1, 2, 3 oder 4 C-Atomen oder Cycloalkyl mit 3, 4, 5 oder 6 C-Atomen, in dem 1, 2, 3 oder 4 Wasserstoff-Atome durch Fluoratome ersetzt sein können;
R9 und R10 unabhängig voneinander Wasserstoff, Methyl, Ethyl oder -CH₂-CF₃;
oder
R3 Phenyl oder -O-Phenyl,
in denen die Phenylreste unsubstituiert sind oder substituiert sind mit 1, 2 oder 3 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
R4 Wasserstoff, F, Cl, -CN, -SO₂CH₃ oder Methyl;
R5 und R6 unabhängig voneinander Wasserstoff, Methyl oder Ethyl;
R7 und R8 unabhängig voneinander Wasserstoff, Alkyl mit 1, 2, 3 oder 4 C-Atomen, Cycloalkyl mit 3, 4, 5 oder 6 C-Atomen, -CF₃ oder Phenyl, wobei der Phenylrest unsubstituiert ist oder substituiert ist mit 1, 2 oder 3 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
oder
R7 und R8 bilden zusammen mit dem sie tragenden C-Atom ein Cycloalkyl mit 3, 4, 5 oder 6 C-Atomen;
X -CH₂- oder -NR17-
R17 Wasserstoff, Methyl, Ethyl, Cycloalkyl mit 3, 4, 5 oder 6 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, I, -O-CF₃, -O-CH₂-CF₃, -S-CF₃, Methoxy, Ethoxy, Alkyl mit 1, 2, 3 oder 4 C-Atomen und -SO₂CH₃;
Y eine Bindung oder -CH₂-;
sowie deren pharmazeutisch verträgliche Salze.

3. Verbindungen der Formel I nach Anspruch 1 oder 2, in denen bedeuten:
R1 Wasserstoff, Methyl, Ethyl, Methoxy, Ethoxy, F, Cl, -O-CF₃, -O-CH₂-CF₃ oder -S-CF3;
R2 Wasserstoff, F, Cl, -O-CF₃, -O-CH₂-CF₃ oder -S-CF₃;
R3 F, Cl, -CN, -SO₂CH₃, -CF₃, -O-CF₃, -O-CH₂-CF₃, -SO₂CF₃, -S-CF₃, Alkyl mit 1, 2, 3 oder 4 C-Atomen oder Cycloalkyl mit 3, 4, 5 oder 6 C-Atomen, in dem 1, 2, 3 oder 4 Wasserstoff-Atome durch Fluoratome ersetzt sein können;
oder
R3 Phenyl oder -O-Phenyl,
in denen die Phenylreste unsubstituiert sind oder substituiert sind mit 1oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
R4 Wasserstoff oder F;
R5 und R6 unabhängig voneinander Wasserstoff oder Methyl;
R7 und R8 unabhängig voneinander Wasserstoff, Alkyl mit 1, 2, 3 oder 4 C-Atomen, Cycloalkyl mit 3, 4, 5 oder 6 C-Atomen, -CF₃ oder Phenyl, wobei der Phenylrest unsubstituiert ist oder substituiert ist mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
oder
R7 und R8 bilden zusammen mit dem sie tragenden C-Atom ein Cycloalkyl mit 3, 4, 5 oder 6 C-Atomen;
X -CH₂- oder -NR17-
R17 Wasserstoff, Methyl, Ethyl, Cycloalkyl mit 3, 4, 5 oder 6 C-Atomen, -CH₂-CF₃ oder Phenyl, das unsubstituiert ist oder substituiert ist mit 1, 2 oder 3 Resten ausgewählt aus der Gruppe bestehend aus F, Cl, Br, 1, -O-CF₃, -O-CH₂-CF₃, -S-CF₃, Methoxy, Ethoxy, Alkyl mit 1, 2, 3 oder 4 C-Atomen und -SO₂CH₃;
Y eine Bindung oder -CH₂-;
sowie deren pharmazeutisch verträgliche Salze.

4. Verbindungen der Formel I nach einem oder mehreren der Ansprüche 1, 2 oder 3, in denen bedeuten:
R1 Wasserstoff, Methyl -O-CH₂-CF₃, oder -S-CF₃;
R2 Wasserstoff;
R3 -CN, -SO₂CH₃, -CF₃, -O-CF₃, -O-CH₂-CF₃, -SO₂CF₃, -S-CF₃;
R4 Wasserstoff;
R5 und R6 Wasserstoff;
R7 Wasserstoff;
R8 Alkyl mit 1, 2, 3 oder 4 C-Atomen, Cycloalkyl mit 3, 4, 5 oder 6 C-Atomen, -CF₃ oder Phenyl, wobei der Phenylrest unsubstituiert ist oder substituiert ist mit 1 oder 2 Resten ausgewählt aus der Gruppe bestehend aus Reihe F, Cl, -O-CF₃, -O-CH₂-CF₃, -S-CF₃, Methoxy, Ethoxy, Alkyl mit 1, 2, 3 oder 4 C-Atomen und -SO₂CH₃;
X -CH₂-
Y eine Bindung oder -CH₂-;
sowie deren pharmazeutisch verträgliche Salze.

5. Verbindungen der Formel I nach einem oder mehreren der Ansprüche 1, 2 oder 3, in denen bedeuten:
R1 Wasserstoff, Methyl -O-CH₂-CF₃, oder -S-CF₃;
R2 Wasserstoff;
R3 -CN, -SO₂CH₃, -CF₃, -O-CF₃, -O-CH₂-CF₃, -SO₂CF₃, -S-CF₃;
R4 Wasserstoff;
R5 und R6 unabhängig voneinander Wasserstoff oder Methyl;
R7 Wasserstoff;
R8 Wasserstoff,
X -CH₂- oder -NR17-,
R17 Wasserstoff, Methyl, Ethyl, Cycloalkyl mit 3, 4, 5 oder 6 C-Atomen oder -CH₂-CF₃;
Y eine Bindung oder -CH₂-;
sowie deren pharmazeutisch verträgliche Salze.

6. Verbindungen der Formel I nach einem der Ansprüche 1 bis 3 ausgewählt aus:
N-[4-(1,1-Dioxo-isothiazolidin-2-yl)-5-methansulfonyl-2-methyl-benzoyl]-guanidin,
N-[4-(1,1-Dioxo-[1,2]thiazinan-2-yl)-5-methansulfonyl-2-methyl-benzoyl]-guanidin,
N-[4-(3,3-Dimethyl-1, 1-dioxo-isothiazolidin-2-yl)-5-methansulfonyl-2-methyl- benzoyl]-guanidin,
N-[4-(1,1-Dioxo-4-phenyl-isothiazolidin-2-yl)-5-methansulfonyl-2-methyl-benzoyl]-guanidin,
N-{4-[4-(4-Fluor-phenyl)-1,1-dioxo-isothiazolidin-2-yl]-5-methansulfonyl-2-methyl-benzoyl}-guanidin,
N-[4-(1,1-Dioxo-4-phenyl-1-[1,2]thiazinan-2-yl)-5-methansulfonyl-2-methyl- benzoyl]-guanidin,
N-[4-(5-Cyclopropyl-1,1-dioxo-1-[1,2,5]thiadiazolidin-2-yl)-5-methansulfonyl-2-methyl-benzoyl]-guanidin,
N-[4-(6-Cyclopropyl-1,1-dioxo-1-[1,2,6]thiadiazinan-2-yl)-5-methansulfonyl-2-methyl-benzoyl]-guanidin,
N-[4-(1,1-Dioxo-1-isothiazolidin-2-yl)-3-trifluoromethyl-benzoyl]-guanidin,
N-[4-(1,1-Dioxo-1-[1,2]thiazinan-2-yl)-3-trifluoromethyl-benzoyl]-guanidin
und deren pharmazeutisch verträgliche, Salze.

7. Verfahren zur Herstellung einer Verbindung der Formel I nach einem oder mehreren der Ansprüche 1, 2, 3, 4 oder 5 und/oder deren pharmazeutisch verträgliche Salze, **dadurch gekennzeichnet, dass** eine Verbindung der Formel II 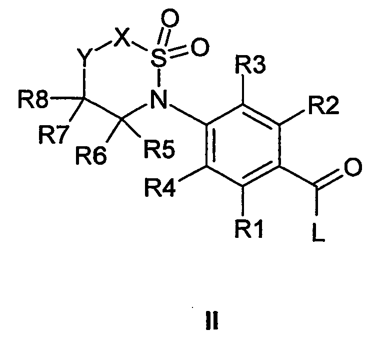 worin R1 bis R8 die Bedeutung wie in Verbindungen der Formel I besitzen und L für eine nucleophil substituierbare Fluchtgruppe steht, mit Guanidin umsetzt wird.

8. Verbindung der Formel II 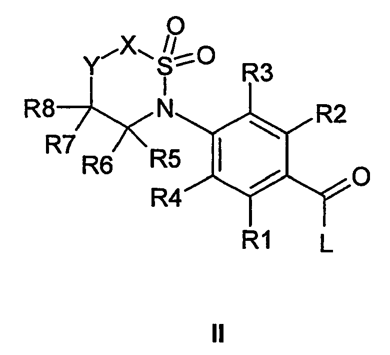 worin R1 bis R8, X und Y wie in den Ansprüchen 1, 2, 3, 4 oder 5 definiert sind und L wie in Anspruch 7 definiert ist,
wobei Verbindungen der Formel II ausgenommen sind, in denen R1, R2, R3 und R4 gleichzeitig Wasserstoff sind und X -CH₂- ist,
und wobei die folgenden Verbindungen ausgeschlossen sind
4-(1,1-Dioxo-isothiazolidin-2-yl)-3-methyl-benzoesäuremethylester,
4-(1, 1-Dioxo-isothiazolidin-2-yl)-3-methyl-benzoesäure,
4-(1,1-Dioxo-1-[1,2,6]thiadiazinan-2-yl)-3-methyl-benzoesäuremethylester,
4-(1,1-Dioxo-1-[1,2,6]thiadiazinan-2-yl)-3-methyl-benzoesäure
und
3-Chlor-4-(1,1-Dioxo-1-[1,2]thiazinan-2-yl)-benzoesäure.

9. Verbindung der Formel I und/oder deren pharmazeutisch verträgliche Salze nach einem oder mehreren der Ansprüche 1 bis 6 zur Verwendung als Medikament.

10. Verwendung einer Verbindung der Formel I und/oder deren pharmazeutisch verträgliche Salze nach einem oder mehreren der Ansprüche 1 bis 6 zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyrcotoxikose oder zur Herstellung eines Diagnostikums.

11. Verwendung einer Verbindung der Formel und/oder deren pharmazeutisch verträgliche Salze nach einem oder mehreren der Ansprüche 1 bis 6 in Kombination mit anderen Arzneimitteln oder Wirkstoffen zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimerns des Herzens, des Herzinfarkts, der Angina Pectoris, zur Behandlung oder Prophylaxe von ischämischen Zuständen des Herzens, von Ischämischen Zuständen des peripheren und zentralen Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der Prostata-Hyperplasle, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothellaler Dysfunktion, von claudicatio intermittens, zur Behandlung oder Prophylaxe fibrotischer Erkrankungen innerer Organe, fibrotischer Erkrankungen der Leber, tibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inflammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlangerung, zur Behandlung und Senkung der cardiotoxischen Wirkungen in der Thyreotoxikose oder zur Herstellung eines Diagnostikums.

12. Verwendung einer Verbindung der Formel I und/oder deren pharmazeutisch verträgliche Salze nach Anspruch 10 in der Kombination mit cardiotoxischen und cytotoxischen Arzneimitteln oder Wirkstoffen zur Herstellung eines Medikaments mit verminderten cardiotoxischen und cytotoxischen Eigenschaften.

13. Verwendung einer Verbindung der Formel I und/oder deren pharmazeutisch verträgliche Salze allein oder in Kombination mit anderen Arzneimitteln oder Wirkstoffen nach Anspruch 9 und/oder 10 zur Herstellung eines Medikaments zur Behandlung oder Prophylaxe von akuten oder chronischen Schäden, Erkrankungen oder indirekten Folgeerkrankungen von Organen und Geweben, die durch Ischämie- oder durch Reperfusionsereignisse verursacht werden.

14. Verwendung einer Verbindung der Formel I und/oder deren pharmazeutisch verträgliche Salze allein oder in Kombination mit anderen Arzneimitteln oder Wirkstoffen nach Anspruch 9 und/oder 10 zur Herstellung eines Medikaments zur Behandlung lebensbedrohlichen Kammerfilmmerns des Herzens.

15. Verwendung einer Verbindung der Formel I und/oder deren pharmazeutisch verträgliche Salze allein oder in Kombination mit anderen Arzneimitteln oder Wirkstoffen nach Anspruch 9 und/oder 10 zur Herstellung eines Medikaments zur Behandlung oder zur Prophylaxe der Metastasierung.

16. Verwendung einer Verbindung der Formel I und/oder deren pharmazeutisch verträgliche Salze allein oder in Kombination mit anderen Arzneimitteln oder Wirkstoffen nach Anspruch 9 und/oder 10 zur Herstellung eines Medikaments zur Behandlung oder Prophylaxe fibrotischer Erkrankungen des Herzens, der Herzinsuffizienz oder des Congestive Heart failure.

17. Heilmittel für die humane, veterinäre und/oder phytoprotektive Anwendung enthaltend eine wirksame Menge einer Verbindung der Formel und/oder deren pharmazeutisch verträgliche Salze nach einem oder mehreren der Ansprüche 1 bis 6, zusammen mit pharmazeutisch annehmbaren Träger- und Zusatzstoffen.

18. Heilmittel für die humane, veterinäre oder phytoprotektive Anwendung enthaltend eine wirksame Menge einer Verbindung der Formel 1 und/oder deren pharmazeutisch verträgliche Salze nach einem oder mehreren der Ansprüche 1 bis 6, zusammen mit pharmazeutisch annehmbaren Träger und Zusatzstoffen in Kombination mit anderen pharmakologischen Wirkstoffen oder Arzneimitteln.

## Claims

1. A compound of the formula I 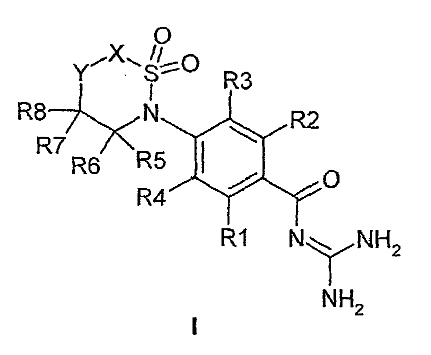 in which the meanings are
R1 hydrogen, alkyl having 1, 2, 3 or 4 carbon atoms, alkoxy having 1, 2, 3 or 4 carbon atoms, F, Cl, Br, I, -CN, NR13R14, -O-(CH₂)ₙ-(CF₂)ₒ-CF₃ or -(SOₘ)_{q} -(CH₂)ᵣ(CF₂)ₛ-CF₃;
R13 and R14 independently of one another hydrogen, alkyl having 1, 2, 3 or 4 carbon atoms or -CH₂-CF₃;
m zero, 1 or 2
n, o, q, r and s independently of one another zero or 1;
R2 hydrogen, alkyl having 1, 2, 3 or 4 carbon atoms, alkoxy having 1, 2, 3 or 4 carbon atoms, F, Cl, Br, I, -CN, NR15R16,
-O-(CH₂)ᵤ-(CF₂)ᵥ-CF₃ or -(SO_{w})ₓ-(CH₂)_{y}-(CF₂)_{z}-CF₃;
R15 and R16 independently of one another hydrogen, alkyl having 1, 2, 3 or 4 carbon atoms or -CH₂-CF₃;
w zero, 1 or 2
u, v, x, y and z independently of one another zero or 1;
R3 hydrogen, F, Cl, Br, I, -CN, -SO₂CH₃, alkoxy having 1, 2, 3 or 4 carbon atoms, NR9R10, -O-(CH₂)_{b}-(CF₂)_{c}-CF₃,
-(SO_{d})ₑ-(CH₂)_{f}-(CF₂)_{g}-CF₃, alkyl having 1, 2, 3, 4, 5 or 6 carbon atoms or cycloalkyl having 3, 4, 5, 6, 7 or 8 carbon atoms, in which 1, 2, 3 or 4 hydrogen atoms may be replaced by fluorine atoms;
R9 and R10 independently of one another hydrogen, alkyl having 1, 2, 3 or 4 carbon atoms or -CH₂-CF₃;
b,c,eandg independently of one another zero or 1;
d zero, 1 or 2;
f zero, 1, 2, 3 or 4;
or
R3 -(CH₂)ₕ-phenyl or --O-phenyl,
in which the phenyl radicals are unsubstituted or are substituted by 1, 2 or 3 radicals selected from the group consisting of series F, Cl, Br, I, -Oⱼ-(CH₂)ₖ-CF₃, alkoxy having 1, 2, 3 or 4 carbon atoms, alkyl having 1, 2, 3 or 4 carbon atoms and -SO₂CH₃;
j zero or 1;
k zero, 1, 2 or 3;
h zero, 1, 2, 3 or 4;
or
R3 -(CH₂)ₐₐ-heteroaryl,
which is unsubstituted or is substituted by 1, 2 or 3 radicals selected from the group consisting of F, Cl, Br, I, -O_{bb}-(CH₂)_{cc}-CF₃, alkoxy having 1, 2, 3 or 4 carbon atoms, alkyl having 1, 2, 3 or 4 carbon atoms and -SO₂CH₃;
bb zero or 1;
cc zero or 1, 2 or 3;
aa zero, 1, 2, 3 or 4;
R4 hydrogen, F, Cl, Br, I, -CN, -SO₂CH₃, alkoxy having 1, 2, 3 or 4 carbon atoms, NR11R12, -O-(CH₂)ₑₑ-(CF₂)_{ff}-CF₃; -(SO_{gg})ₕₕ-(CH₂)ⱼⱼ-(CF₂)ₖₖ-CF₃, alkyl having 1, 2, 3, 4, 5 or 6 carbon atoms or cycloalkyl having 3, 4, 5, 6, 7 or 8 carbon atoms, in which 1, 2, 3 or 4 hydrogen atoms may be substituted by fluorine atoms;
R11 and R12 independently of one another hydrogen, alkyl having 1, 2, 3 or 4 carbon atoms or -CH₂-CF₃;
ee, ff, hh and kk independently of one another zero or 1;
gg zero, 1 or 2;
jj zero, 1, 2, 3 or 4;
or
R4 -(CH₂)ₗₗ-phenyl or -O-phenyl,
in which the phenyl radicals are unsubstituted or are substituted by 1, 2 or 3 radicals selected from the group consisting of series F, Cl, Br, I, -Oₘₘ-(CH₂)ₙₙ-CF₃, alkoxy having 1, 2, 3 or 4 carbon atoms, alkyl having 1, 2, 3 or 4 carbon atoms and -SO₂CH₃;
mm zero or 1;
nn zero, 1, 2 or 3;
II zero, 1, 2, 3 or 4;
or
R4 -(CH₂)ₒₒ-heteroaryl,
which is unsubstituted or is substituted by 1, 2 or 3 radicals selected from the group consisting of F, Cl, Br, I, -Oₚₚ-(CH₂)ᵣᵣ-CF₃, alkoxy having 1, 2, 3 or 4 carbon atoms, alkyl having 1, 2, 3 or 4 carbon atoms and -SO₂CH₃;
pp zero or 1;
rr zero, 1, 2 or 3;
oo zero, 1, 2, 3 or 4;
R5 and R6 independently of one another hydrogen or alkyl having 1, 2, 3 or 4 carbon atoms;
R7 and R8 independently of one another hydrogen, alkyl having 1, 2, 3 or 4 carbon atoms, cycloalkyl having 3, 4, 5, 6, 7 or 8 carbon atoms, -CF₃ or phenyl, where the phenyl radical is unsubstituted or is substituted by 1, 2 or 3 radicals selected from the group consisting of series F, Cl, Br, I, -Oₛₛ-(CH₂)ₜₜ-CF₃, alkoxy having 1, 2, 3 or 4 carbon atoms, alkyl having 1, 2, 3 or 4 carbon atoms and -SO₂CH₃;
ss zero or 1
tt zero, 1, 2 or 3;
or
R7 and R8 form together with the carbon atom carrying them a cycloalkyl having 3, 4, 5, 6, 7 or 8 carbon atoms;
X -CH₂- or -NR17-
R17 hydrogen, alkyl having 1, 2, 3 or 4 carbon atoms, cycloalkyl having 3, 4, 5, 6, 7 or 8 carbon atoms, -CH₂-CF₃ or phenyl which is unsubstituted or is substituted by 1, 2 or 3 radicals selected from the group consisting of F, Cl, Br, I, -Oᵤᵤ-(CH₂)ᵥᵥ-CF₃, alkoxy having 1, 2, 3 or 4 carbon atoms, alkyl having 1, 2, 3 or 4 carbon atoms and -SO₂CH₃;
uu zero or 1;
w zero, 1, 2 or 3;
Y a bond or an alkylene chain having 1, 2 or 3 carbon atoms;
and the pharmaceutically acceptable salts thereof.

2. A compound of the formula I as claimed in claim 1, in which the meanings are:
R1 hydrogen, alkyl having 1, 2, 3 or 4 carbon atoms, alkoxy having 1, 2, 3 or 4 carbon atoms, F, Cl, NR13R14, -O-(CH₂)ₙ-(CF₂)ₒ-CF₃ or -(SOₘ)_{q}-(CH₂)ᵣ-(CF₂)ₛ-CF₃;
R13 and R14 independently of one another hydrogen, methyl, ethyl or CH₂-CF₃;
m zero, 1 or 2
n, o, q, r and s independently of one another zero or 1;
R2 hydrogen, methyl, methoxy, F, Cl, -O-CF₃, -O-CH₂-CF₃ or -S-CF₃;
R3 hydrogen, F, Cl, -CN, -SO₂CH₃, methoxy, ethoxy, NR9R10, -O-CF₃, -O-CH₂-CF₃, -SO₂CF₃, -S-CF₃, -CF₃, alkyl having 1, 2, 3 or 4 carbon atoms or cycloalkyl having 3, 4, 5 or 6 carbon atoms, in which 1, 2, 3 or 4 hydrogen atoms may be substituted by fluorine atoms;
R9 and R10 independently of one another hydrogen, methyl, ethyl or -CH₂-CF₃;
or
R3 phenyl or -O-phenyl,
in which the phenyl radicals are unsubstituted or are substituted by 1, 2 or 3 radicals selected from the group consisting of series F, Cl, -O-CF₃, -O-CH₂-CF₃, -S-CF₃, methoxy, ethoxy, alkyl having 1, 2, 3 or 4 carbon atoms and -SO₂CH₃;
R4 hydrogen, F, Cl, -CN, -SO₂CH₃ or methyl;
R5 and R6 independently of one another hydrogen, methyl or ethyl;
R7 and R8 independently of one another hydrogen, alkyl having 1, 2, 3 or 4 carbon atoms, cycloalkyl having 3, 4, 5 or 6 carbon atoms, -CF₃ or phenyl, where the phenyl radical is unsubstituted or is substituted by 1, 2 or 3 radicals selected from the group consisting of series F, Cl, -O-CF₃, -O-CH₂-CF₃, -S-CF₃, methoxy, ethoxy, alkyl having 1, 2, 3 or 4 carbon atoms and -SO₂CH₃;
or
R7 and R8 form together with the carbon atom carrying them a cycloalkyl having 3, 4, 5 or 6 carbon atoms;
X -CH₂- or -NR17-
R17 hydrogen, methyl, ethyl, cycloalkyl having 3, 4, 5 or 6 carbon atoms, -CH₂-CF₃ or phenyl which is unsubstituted or is substituted by 1, 2 or 3 radicals selected from the group consisting of F, Cl, Br, I, -O-CF₃, -O-CH₂-CF₃, -S-CF₃, methoxy, ethoxy, alkyl having 1, 2, 3 or 4 carbon atoms and -SO₂CH₃;
Y a bond or -CH₂-;
and the pharmaceutically acceptable salts thereof.

3. A compound of the formula I as claimed in claim 1 or 2, in which the meanings are:
R1 hydrogen, methyl, ethyl, methoxy, ethoxy, F, Cl, -O-CF₃, -O-CH₂-CF₃ or -S-CF₃;
R2 hydrogen, F, Cl, -O-CF₃, -O-CH₂-CF₃ or -S-CF₃;
R₃ F, Cl, -CN, -SO₂CH₃, -CF₃, -O-CF₃, -O-CH₂-CF₃, -SO₂CF₃, -S-CF₃, alkyl having 1, 2, 3 or 4 carbon atoms or cycloalkyl having 3, 4, 5 or 6 carbon atoms, in which 1, 2, 3 or 4 hydrogen atoms may be replaced by fluorine atoms;
or
R3 phenyl or -O-phenyl,
in which the phenyl radicals are unsubstituted or are substituted by 1 or 2 radicals selected from the group consisting of series F, Cl, -O-CF₃, -O-CH₂-CF₃, -S-CF₃, methoxy, ethoxy, alkyl having 1, 2, 3 or 4 carbon atoms and -SO₂CH₃;
R4 hydrogen or F;
R5 and R6 independently of one another hydrogen or methyl;
R7 and R8 independently of one another hydrogen, alkyl having 1, 2, 3 or 4 carbon atoms, cycloalkyl having 3, 4, 5 or 6 carbon atoms, -CF₃ or phenyl, where the phenyl radical is unsubstituted or is substituted by 1 or 2 radicals selected from the group consisting of series F, Cl, -O-CF₃, -O-CH₂-CF₃, -S-CF₃, methoxy, ethoxy, alkyl having 1, 2, 3 or 4 carbon atoms and -SO₂CH₃;
or
R7 and R8 form together with the carbon atom carrying them a cycloalkyl having 3, 4, 5 or 6 carbon atoms;
X -CH₂- or -NR17-
R17 hydrogen, methyl, ethyl, cycloalkyl having 3, 4, 5 or 6 carbon atoms, -CH₂-CF₃ or phenyl which is unsubstituted or substituted by 1, 2 or 3 radicals selected from the group consisting of F, Cl, Br, I, -O-CF₃, -O-CH₂-CF₃, -S-CF₃, methoxy, ethoxy, alkyl having 1, 2, 3 or 4 carbon atoms and -SO₂CH₃;
Y a bond or -CH₂-;
and the pharmaceutically acceptable salts thereof.

4. A compound of the formula I as claimed in one or more of claims 1, 2 or 3, in which the meanings are:
R1 hydrogen, methyl, -O-CH₂-CF₃ or -S-CF₃;
R2 hydrogen;
R3 -CN, -SO₂CH₃, -CF₃, -O-CF₃, -O-CH₂-CF₃, -SO₂CF₃, -S-CF₃;
R4 hydrogen;
R5 and R6 hydrogen;
R7 hydrogen;
R8 alkyl having 1, 2, 3 or 4 carbon atoms, cycloalkyl having 3, 4, 5 or 6 carbon atoms, -CF₃ or phenyl, where the phenyl radical is unsubstituted or is substituted by 1 or 2 radicals selected from the group consisting of series F, Cl, -O-CF₃, -O-CH₂-CF₃, -S-CF₃, methoxy, ethoxy, alkyl having 1, 2, 3 or 4 carbon atoms and -SO₂CH₃;
X -CH₂-
Y a bond or -CH₂-;
and the pharmaceutically acceptable salts thereof.

5. A compound of the formula I as claimed in one or more of claims 1, 2 or 3, in which the meanings are:
R1 hydrogen, methyl, -O-CH₂-CF₃ or -S-CF₃;
R2 hydrogen;
R3 -CN, -SO₂CH₃, -CF₃, -O-CF₃, -O-CH₂-CF₃, -SO₂CF₃, -S-CF₃;
R4 hydrogen;
R5 and R6 independently of one another hydrogen or methyl;
R7 hydrogen;
R8 hydrogen;
X -CH₂- or -NR17-
R17 hydrogen, methyl, ethyl, cycloalkyl having 3, 4, 5 or 6 carbon atoms or -CH₂-CF₃;
Y a bond or -CH₂-;
and the pharmaceutically acceptable salts thereof.

6. A compound of the formula I as claimed in any of claims 1 to 3 selected from:
N-[4-(1,1-dioxoisothiazolidin-2-yl)-5-methanesulfonyl-2-methylbenzoyl]-guanidine,
N-[4-(1,1-dioxo-[1,2]thiazinan-2-yl)-5-methanesulfonyl-2-methylbenzoyl]-guanidine,
N-[4-(3,3-dimethyl-1,1-dioxoisothiazolidin-2-yl)-5-methanesulfonyl-2-methylbenzoyl]guanidine,
N-[4-(1,1-dioxo-4-phenylisothiazolidin-2-yl)-5-methanesulfonyl-2-methylbenzoyl]guanidine,
N-{4-[4-(4-fluorophenyl)-1,1-dioxoisothiazolidin-2-yl]-5-methanesulfonyl-2-methylbenzoyl}guanidine,
N-[4-(1,1-dioxo-4-phenyl-1-[1,2]thiazinan-2-yl)-5-methanesulfonyl-2-methylbenzoyl]guanidine,
N-[4-(5-cyclopropyl-1,1-dioxo-1-[1,2,5]thiadiazolidin-2-yl)-5-methanesulfonyl-2-methylbenzoyl]guanid ine,
N-[4-(6-cyclopropyl-1,1-dioxo-1-[1,2,6]thiadiazinan-2-yl)-5-methanesulfonyl-2-methylbenzoyl]guanidine,
N-[4-(1,1-dioxo-1-isothiazolidin-2-yl)-3-trifluoromethylbenzoyl]guanidine,
N-[4-(1,1-dioxo-1-[1,2]thiazinan-2-yl)-3-trifluoromethylbenzoyl]guanidine
and the pharmaceutically acceptable salts thereof.

7. A process for preparing a compound of the formula I as claimed in one or more of claims 1, 2, 3, 4 or 5 and/or the pharmaceutically acceptable salts thereof, which comprises reacting a compound of the formula II 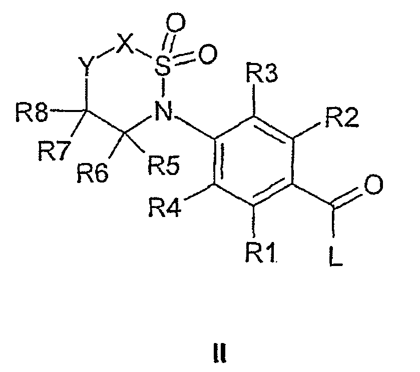 in which R1 to R8 have the meaning as in compounds of the formula I, and L is a leaving group which can undergo nucleophilic substitution, with guanidine.

8. A compound of the formula II 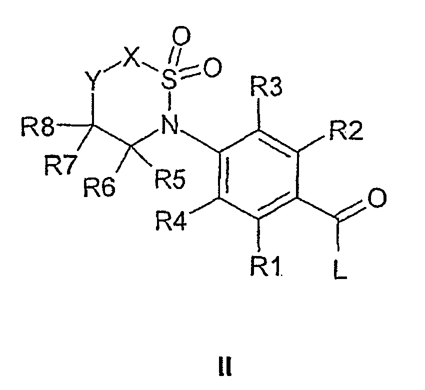 in which R1 to R8, X and Y are as defined in claims 1, 2, 3, 4 or 5, and L is as defined in claim 7,
excepting compounds of the formula II in which R1, R2, R3 and R4 are simultaneously hydrogen, and X is -CH₂-,
and excluding the following compounds
methyl 4-(1,1-dioxoisothiazolidin-2-yl)-3-methylbenzoate,
4-(1,1-dioxoisothiazolidin-2-yl)-3-methylbenzoic acid,
methyl 4-(1,1-dioxo-1-[1,2,6]thiadiazinan-2-yl)-3-methylbenzoate,
4-(1,1-dioxo-1-[1,2,6]thiadiazinan-2-yl)-3-methylbenzoic acid
and
3-chloro-4-(1,1-dioxo-1-[1,2]thiazinan-2-yl)benzoic acid.

9. A compound of the formula I and/or the pharmaceutically acceptable salts thereof as claimed in one or more of claims 1 to 6 for use as medicament.

10. The use of a compound of the formula I and/or the pharmaceutically acceptable salts thereof as claimed in one or more of claims 1 to 6 for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria and of coccidiosis in poultry, and for use for surgical operations and organ transplantations, for preserving and storing transplants for surgical procedures, for use in bypass operations, for use in resuscitation after a cardiac arrest, for preventing age-related tissue change, for producing a medicament directed against aging or for prolonging life, for the treatment and reduction of the cardiotoxic effects in thyrotoxicosis or for producing a diagnostic aid.

11. The use of a compound of the formula I and/or the pharmaceutically acceptable salts thereof as claimed in one or more of claims 1 to 6 in combination with other medicaments or active ingredients for producing a medicament for the treatment or prophylaxis of acute or chronic damage, disorders or indirect sequelae of organs and tissues caused by ischemic or reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudication, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria and of coccidiosis in poultry, and for use for surgical operations and organ transplantations, for preserving and storing transplants for surgical procedures, for use in bypass operations, for use in resuscitation after a cardiac arrest, for preventing age-related tissue change, for producing a medicament directed against aging or for prolonging life, for the treatment and reduction of the cardiotoxic effects in thyrotoxicosis or for producing a diagnostic aid.

12. The use of a compound of the formula I and/or the pharmaceutically acceptable salts thereof as claimed in claim 10 in combination with cardiotoxic and cytotoxic medicaments or active ingredients for producing a medicament with reduced cardiotoxic and cytotoxic properties.

13. The use of a compound of the formula I and/or the pharmaceutically acceptable salts thereof alone or in combination with other medicaments or active ingredients as claimed in claim 9 and/or 10 for producing a medicament for the treatment or prophylaxis of acute or chronic damage, disorders or indirect sequelae of organs and tissues caused by ischemic or reperfusion events.

14. The use of a compound of the formula I and/or the pharmaceutically acceptable salts thereof alone or in combination with other medicaments or active ingredients as claimed in claim 9 and/or 10 for producing a medicament for the treatment of life-threatening cardiac ventricular fibrillation.

15. The use of a compound of the formula I and/or the pharmaceutically acceptable salts thereof alone or in combination with other medicaments or active ingredients as claimed in claim 9 and/or 10 for producing a medicament for the treatment or prophylaxis of metastasis.

16. The use of a compound of the formula I and/or the pharmaceutically acceptable salts thereof alone or in combination with other medicaments or active ingredients as claimed in claim 9 and/or 10 for producing a medicament for the treatment or prophylaxis of fibrotic disorders of the heart, of heart failure or of congestive heart failure.

17. A medicine for human, veterinary and/or phytoprotective use comprising an effective amount of a compound of the formula I and/or the pharmaceutically acceptable salts thereof as claimed in one or more of claims 1 to 6, together with pharmaceutically acceptable carriers and additives.

18. A medicine for human, veterinary or phytoprotective use comprising an effective amount of a compound of the formula I and/or the pharmaceutically acceptable salts thereof as claimed in one or more of claims 1 to 6, together with pharmaceutically acceptable carriers and additives in combination with other pharmacological active ingredients or medicaments.

## Revendications

1. Composés de formule I 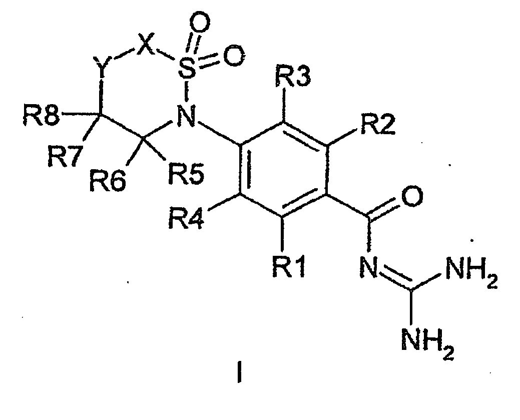 dans laquelle
R1 représente un atome d'hydrogène, un groupe alkyle ayant 1, 2, 3 ou 4 atomes de carbone, alcoxy ayant 1, 2, 3 ou 4 atomes de carbone, F, Cl, Br, I, -CN, NR13R14, -O-(CH₂)ₙ-(CF₂)ₒ-CF₃ ou -(SOₘ)_{q}-(CH₂)ᵣ-(CF₂)ₛ-CF₃ ;
R13 et R14 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2 ;
n, o, q, r et s représentent, indépendamment les uns des autres, zéro ou 1 ;
R2 représente un atome d'hydrogène, un groupe alkyle ayant 1, 2, 3 ou 4 atomes de carbone, alcoxy ayant 1, 2, 3 ou 4 atomes de carbone, F, Cl, Br, I, -CN, NR15R16, -O-(CH₂)ᵤ-(CF₂)ᵥ-CF₃ ou -(SO_{w})ₓ-(CH₂)_{y}-(CF₂)_{z}-CF₃ ;
R15 et R16 représentent, indépendamment l'un de l'autre, un atome d'hydrogène, un groupe alkyle ayant 1, 2, 3 ou 4 atomes de carbone ou -CH₂-CF₃ ;
w est zéro, 1 ou 2 ;
u, v, x, y et z représentent, indépendamment les uns des autres, zéro ou 1 ;
R3 représente un atome d'hydrogène, F, Cl, Br, I, -CN, -SO₂CH₃, un groupe alcoxy ayant 1, 2, 3 ou 4 atomes de carbone, NR9R10, -O-(CH₂)_{b}-(CF₂)_{c}-CF₃, -(SO_{d})ₑ-(CH₂)_{f}-(CF₂)_{g}-CF₃, un groupe alkyle ayant 1, 2, 3, 4, 5 ou 6 atomes de carbone ou cycloalkyle ayant 3, 4, 5, 6, 7 ou 8 atomes de carbone, dans lequel 1, 2, 3 ou 4 atomes d'hydrogène peuvent être remplacés par des atomes de fluor ;
R9 et R10 représentent, indépendamment l'un de l'autre, un atome d'hydrogène, un groupe alkyle ayant 1, 2, 3 ou 4 atomes de carbone ou -CH₂-CF₃ ;
b, c, e et g représentent chacun indépendamment zéro ou 1 ;
d est zéro, 1 ou 2 ;
f est zéro, 1, 2, 3 ou 4 ;
ou
R3 représente un groupe -(CH₂)ₕ-phényle ou un groupe -O-phényle,
dans lesquels les radicaux phényle sont non substitués ou sont substitués par 1, 2 ou 3 radicaux choisis dans l'ensemble constitué par F, Cl, Br, I et par les groupes -Oⱼ-(CH₂)ₖ-CF₃, alcoxy ayant 1, 2, 3 ou 4 atomes de carbone, alkyle ayant 1, 2, 3 ou 4 atomes de carbone et -SO₂CH₃ ;
j est zéro ou 1 ;
k est zéro, 1, 2 ou 3 ;
h est zéro, 1, 2, 3 ou 4 ;
ou
R3 représente un groupe -(CH₂)ₐₐ-hétéroaryle qui est non substitué ou est substitué par 1, 2 ou 3 radicaux choisis dans l'ensemble constitué par F, Cl, Br, I et par les groupes -O_{bb}-(CH₂)_{cc}-CF₃, alcoxy ayant 1, 2, 3 ou 4 atomes de carbone, alkyle ayant 1, 2, 3 ou 4 atomes de carbone et -SO₂CH₃ ;
bb est zéro ou 1 ;
cc est zéro, 1, 2 ou 3 :
aa est zéro, 1, 2, 3 ou 4 ;
R4 représente un atome d'hydrogène, F, Cl, Br, I, -CN, -SO₂CH₃, un groupe alcoxy ayant 1, 2, 3 ou 4 atomes de carbone, NR11R12, -O-(CH₂)ₑₑ-(CF₂)_{ff}-CF₃, (SO_{gg})ₕₕ-(CH₂)ⱼⱼ-(CF₂)ₖₖ-CF₃, un groupe alkyle ayant 1, 2, 3, 4, 5 ou 6 atomes de carbone ou cycloalkyle ayant 3, 4, 5, 6, 7 ou 8 atomes de carbone, dans lequel 1, 2, 3 ou 4 atomes d'hydrogène peuvent être remplacés par des atomes de fluor ;
R11 et R12 représentent, indépendamment l'un de l'autre, un atome d'hydrogène, un groupe alkyle ayant 1, 2, 3 ou 4 atomes de carbone ou -CH₂-CF₃ ;
ee, ff, hh et kk représentent chacun indépendamment zéro ou 1 ;
gg est zéro, 1 ou 2 ;
jj est zéro, 1, 2, 3 ou 4 ;
ou
R4 représente un groupe -(CH₂)ₗₗ-phényle ou un groupe -O-phényle,
dans lesquels les radicaux phényle sont non substitués ou sont substitués par 1, 2 ou 3 radicaux choisis dans l'ensemble constitué par F, Cl, Br, I et par les groupes -Oₘₘ-(CH₂)ₙₙ-CF₃, alcoxy ayant 1, 2, 3 ou 4 atomes de carbone, alkyle ayant 1, 2, 3 ou 4 atomes de carbone et -SO₂CH₃ ;
mm est zéro ou 1 ;
nn est zéro, 1, 2 ou 3 ;
11 est zéro, 1, 2, 3 ou 4 ;
ou
R4 représente un groupe -(CH₂)ₒₒ-hétéroaryle qui est non substitué ou est substitué par 1, 2 ou 3 radicaux choisis dans l'ensemble constitué par F, Cl, Br, I et par les groupes -Oₚₚ-(CH₂)ᵣᵣ-CF₃, alcoxy ayant 1, 2, 3 ou 4 atomes de carbone, alkyle ayant 1, 2, 3 ou 4 atomes de carbone et -SO₂CH₃ ;
pp est zéro ou 1 ;
rr est zéro, 1, 2 ou 3 :
oo est zéro, 1, 2, 3 ou 4 ;
R5 et R6 représentent, indépendamment l'un de l'autre, un atome d'hydrogène ou un groupe alkyle ayant 1, 2, 3 ou 4 atomes de carbone ;
R7 et R8 représentent, indépendamment l'un de l'autre, un atome d'hydrogène, un groupe alkyle ayant 1, 2, 3 ou 4 atomes de carbone, cycloalkyle ayant 3, 4, 5, 6, 7 ou 8 atomes de carbone, -CF₃ ou phényle, le radical phényle étant non substitué ou étant substitué par 1, 2 ou 3 radicaux choisis dans l'ensemble constitué par F, Cl, Br, I, -Oₛₛ-(CH₂)ₜₜ-CF₃, alcoxy ayant 1, 2, 3 ou 4 atomes de carbone, alkyle ayant 1, 2, 3 ou 4 atomes de carbone et -SO₂CH₃ ;
ss est zéro ou 1 ;
tt est zéro, 1, 2 ou 3 ;
ou
R7 et R8 forment ensemble, avec l'atome de carbone qui les porte, un groupe cycloalkyle ayant 3, 4, 5, 6, 7 ou 8 atomes de carbone ;
X représente -CH₂ ou -NR17-
R17 représente un atome d'hydrogène, un groupe alkyle ayant 1, 2, 3 ou 4 atomes de carbone, cycloalkyle ayant 3, 4, 5, 6, 7 ou 8 atomes de carbone, -CH₂-CF₃ ou phényle, qui est non substitué ou est substitué par 1, 2 ou 3 radicaux choisis dans l'ensemble constitué par F, Cl, Br, I , -Oᵤᵤ-(CH₂)ᵥᵥ-CF₃, alcoxy ayant 1, 2, 3 ou 4 atomes de carbone, alkyle ayant 1, 2, 3 ou 4 atomes de carbone et -SO₂CH₃ ;
uu est zéro ou 1 ;
vv est zéro, 1, 2 ou 3 ;
Y représente une liaison ou une chaîne alkylène ayant 1, 2 ou 3 atomes de carbone ;
et sels pharmaceutiquement acceptables de tels composés.

2. Composés de formule I selon la revendication 1,
dans lesquels :
R1 représente un atome d'hydrogène, un groupe alkyle ayant 1, 2, 3 ou 4 atomes de carbone, alcoxy ayant 1, 2, 3 ou 4 atomes de carbone, F, Cl, NR13R14, -O-(CH₂)ₙ-(CF₂)ₒ-CF₃ ou -(SOₘ)_{q}-(CH₂)ᵣ-(CF₂)ₛ-CF₃ ;
R13 et R14 représentent, indépendamment l'un de l'autre, un atome d'hydrogène, un groupe méthyle, éthyle ou -CH₂-CF₃ ;
m est zéro, 1 ou 2
n, o, q, r et s représentent, indépendamment les uns des autres, zéro ou 1 ;
R2 représente un atome d'hydrogène, un groupe méthyle, méthoxy, F, Cl, -O-CF₃, -O-CH₂-CF₃ ou -S-CF₃ ;
R3 représente un atome d'hydrogène, F, Cl, -CN, -SO₂CH₃, un groupe méthoxy, éthoxy, NR9R10, -O-CF₃, -O-CH₂-CF₃, -SO₂CF₃, -S-CF₃, -CF₃, un groupe alkyle ayant 1, 2, 3 ou 4 atomes de carbone ou cycloalkyle ayant 3, 4, 5 ou 6 atomes de carbone, dans lequel 1, 2, 3 ou 4 atomes d'hydrogène peuvent être remplacés par des atomes de fluor ;
R9 et R10 représentent, indépendamment l'un de l'autre, un atome d'hydrogène, un groupe méthyle, éthyle ou -CH₂-CF₃ ;
ou
R3 représente un groupe phényle ou un groupe -O-phényle,
dans lesquels les radicaux phényle sont non substitués ou sont substitués par 1, 2 ou 3 radicaux choisis dans l'ensemble constitué par F, Cl, -O-CF₃, -O-CH₂-CF₃, -S-CF₃, méthoxy, éthoxy, alkyle ayant 1, 2, 3 ou 4 atomes de carbone et -SO₂CH₃ ;
R4 représente un atome d'hydrogène, F, Cl, -CN, -SO₂CH₃ ou méthyle ;
R5 et R6 représentent, indépendamment l'un de l'autre, un atome d'hydrogène ou un groupe méthyle ou éthyle ;
R7 et R8 représentent, indépendamment l'un de l'autre, un atome d'hydrogène, un groupe alkyle ayant 1, 2, 3 ou 4 atomes de carbone, cycloalkyle ayant 3, 4, 5 ou 6 atomes de carbone, -CF₃ ou phényle, le radical phényle étant non substitué ou étant substitué par 1, 2 ou 3 radicaux choisis dans l'ensemble constitué par F, Cl , -O-CF₃, -O-CH₂-CF₃, -S-CF₃, méthoxy, éthoxy, alkyle ayant 1, 2, 3 ou 4 atomes de carbone et -SO₂CH₃ ;
ou
R7 et R8 forment ensemble, avec l'atome de carbone qui les porte, un groupe cycloalkyle ayant 3, 4, 5 ou 6 atomes de carbone ;
X représente -CH₂ ou -NR17-
R17 représente un atome d'hydrogène, un groupe méthyle, éthyle, cycloalkyle ayant 3, 4, 5 ou 6 atomes de carbone, -CH₂-CF₃ ou phényle, qui est non substitué ou est substitué par 1, 2 ou 3 radicaux choisis dans l'ensemble constitué par F, Cl, Br, I, -O-CF₃, -O-CH₂-CF₃, -S-CF₃, méthoxy, éthoxy, alkyle ayant 1, 2, 3 ou 4 atomes de carbone et -SO₂CH₃ ;
Y représente une liaison ou -CH₂- ;
et sels pharmaceutiquement acceptables de tels composés.

3. Composés de formule I selon la revendication 1 ou 2, dans lesquels :
R1 représente un atome d'hydrogène, un groupe méthyle, éthyle, méthoxy, éthoxy, F, Cl, -O-CF₃, -O-CH₂-CF₃ ou -S-CF₃ ;
R2 représente un atome d'hydrogène, F, Cl, -O-CF₃-, -O-CH₂-CF₃ ou -S-CF₃ ;
R3 représente F, Cl, -CN, -SO₂CH₃, -CF₃, -O-CF₃, -O-CH₂-CF₃, -SO₂CF₃, -S-CF₃ ; un groupe alkyle ayant 1, 2, 3 ou 4 atomes de carbone ou cycloalkyle ayant 3, 4, 5 ou 6 atomes de carbone, dans lequel 1, 2, 3 ou 4 atomes d'hydrogène peuvent être remplacés par des atomes de fluor ;
ou
R3 représente un groupe phényle ou un groupe O-phényle
dans lesquels les groupes phényle sont non substitués ou sont substitués par 1 ou 2 radicaux choisis dans l'ensemble constitué par F, cl, -O-CF₃, -O-CH₂-CF₃, -S-CF₃, méthoxy, éthoxy, alkyle ayant 1, 2, 3 ou 4 atomes de carbone et -SO₂CH₃;
R4 représente un atome d'hydrogène ou F ;
R5 et R6 représentent, indépendamment l'un de l'autre, un atome d'hydrogène ou un groupe méthyle ;
R7 et R8 représentent, indépendamment l'un de l'autre, un atome d'hydrogène, un groupe alkyle ayant 1, 2, 3 ou 4 atomes de carbone, cycloalkyle ayant 3, 4, 5 ou 6 atomes de carbone, -CF₃ ou phényle, le radical phényle étant non substitué ou étant substitué par 1 ou 2 radicaux choisis dans l'ensemble constitué par F, Cl, -O-CF₃, -O-CH₂-CF₃, -S-CF₃, méthoxy, éthoxy, alkyle ayant 1, 2, 3 ou 4 atomes de carbone et -SO₂CH₃ ;
ou
R7 et R8 forment ensemble, avec l'atome de carbone qui les porte, un groupe cycloalkyle ayant 3, 4, 5 ou 6 atomes de carbone ;
X représente -CH₂ ou -NR17-
R17 représente un atome d'hydrogène, un groupe méthyle, éthyle, cycloalkyle ayant 3, 4, 5 ou 6 atomes de carbone, -CH₂-CF₃ ou phényle, qui est non substitué ou est substitué par 1, 2 ou 3 radicaux choisis dans l'ensemble constitué par F, Cl, Br, I, -O-CF₃, -O-CH₂-CF₃, -S-CF₃, méthoxy, éthoxy, alkyle ayant 1, 2, 3 ou 4 atomes de carbone et -SO₂CH₃ ;
Y représente une liaison ou -CH₂- ;
et sels pharmaceutiquement acceptables de tels composés.

4. Composés de formule I selon une ou plusieurs des revendications 1, 2 ou 3, dans lesquels :
R1 représente un atome d'hydrogène, un groupe méthyle, -O-CH₂-CF₃ ou -S-CF₃ ;
R2 représente un atome d'hydrogène ;
R3 représente -CN, -SO₂CH₃, -CF₃ , -O-CF₃ , -O-CH₂-CF₃, -SO₂CF₃, -S-CF₃;
R4 représente un atome d'hydrogène;
R5 et R6 représentent un atome d'hydrogène ;
R7 représente un atome d'hydrogène ;
R8 représente un groupe alkyle ayant 1, 2, 3 ou 4 atomes de carbone, cycloalkyle ayant 3, 4, 5 ou 6 atomes de carbone, -CF₃ ou phényle, le radical phényle étant non substitué ou étant substitué par 1 ou 2 radicaux choisis dans l'ensemble constitué par F, Cl, -O-CF₃, -O-CH₂-CF₃, -S-CF₃, méthoxy, éthoxy, alkyle ayant 1, 2, 3 ou 4 atomes de carbone et -SO₂CH₃ ;
X représente -CH₂- ;
Y représente une liaison ou -CH₂- ;
et sels pharmaceutiquement acceptables de tels composés.

5. Composés de formule I selon une ou plusieurs des revendications 1, 2 et 3, dans lesquels .
R1 représente un atome d'hydrogène, un groupe méthyle, -O-CH₂-CF₃ ou -S-CF₃ ;
R2 représente un atome d'hydrogène ;
R3 représente -CN, -SO₂CH₃, -CF₃, -O-CF₃, -O-CH₂-CF₃, -SO₂CF₃, -S-CF₃ ;
R4 représente un atome d'hydrogène ;
R5 et R6 représentent, indépendamment l'un de l'autre, un atome d'hydrogène ou le groupe méthyle ;
R7 représente un atome d'hydrogène ;
R8 représente un atome d'hydrogène ;
X représente -CH₂- ou -NR17-,
R17 représente un atome d'hydrogène, un groupe méthyle, éthyle, cycloalkyle ayant 3, 4, 5 ou 6 atomes de carbone ou -CH₂-CF₃ ;
Y représente une liaison ou -CH₂- ;
et sels pharmaceutiquement acceptables de tels composés.

6. Composés de formule I selon l'une quelconque des revendications 1 à 3, choisis parmi :
la N-[4-(1,1-dioxo-isothiazolidin-2-yl)-5-méthane-sulfonyl-2-méthyl-benzoyl]-guanidine,
la N-[4-(1,1-dioxo-[1,2]thiazinan-2-yl)-5-méthane-sulfonyl-2-méthyl-benzoyl]-guanidine,
la N-[4-(3,3-diméthyl-1,1-dioxo-isothiazolidin-2-yl)-5-méthanesulfonyl-2-méthyl-benzoyl]-guanidine,
la N-[4-(1,1-dioxo-4-phényl-isothiazolidin-2-yl)-5-méthanesulfonyl-2-méthyl-benzoyl]-guanidine,
la N-{4-[4-(4-fluoro-phényl)-1,1-dioxo-isothiazolidin-2-yl]-5-méthanesulfonyl-2-méthyl-benzoyl}-guanidine,
la N-[4-(1,1-dioxo-4-phényl-1-[1,2]thiazan-2-yl)-5-méthanesulfonyl-2-méthyl-benzoyl]-guanidine,
la N-[4-(5-cyclopropyl-1,1-dioxo-1-[1,2,5]-thiadiazolidin-2-yl)-5-méthanesulfonyl-2-méthyl-benzoyl]-guanidine,
la N-[4-(6-cyclopropyl-1,1-dioxo-1-[1,2,6]-thiadiazinan-2-yl)-5-méthanesulfonyl-2-méthyl-benzoyl]-guanidine,
la N-[4-(1,1-dioxo-1-isothiazolidin-2-yl)-3-trifluorométhyl-benzoyl]-guanidine,
la N-[4-(1,1-dioxo-1-[1,2]thiazinan-2-yl)-3-trifluorométhyl-benzoyl]-guanidine
et leurs sels pharmaceutiquement acceptables.

7. Procédé pour la préparation d'un composé de formule I selon une ou plusieurs des revendications 1, 2, 3, 4 ou 5, et/ou de ses sels pharmaceutiquement acceptables, **caractérisé en ce qu'**on fait réagir avec de la guanidine un composé de formule II 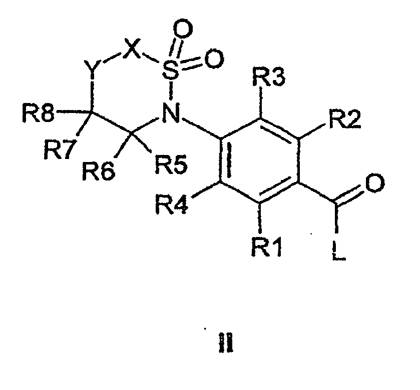 dans laquelle R1 à R8 ont les mêmes significations que dans les composés de formule I et L représente un groupe partant pouvant être remplacé par substitution nucléophile.

8. Composé de formule II 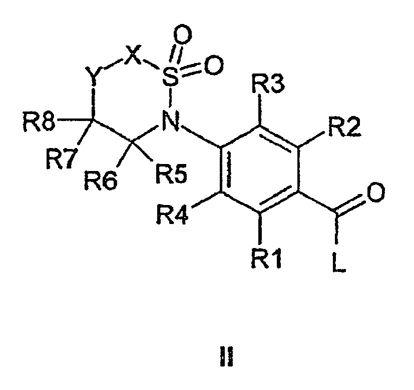 dans laquelle R1 à R8, X et Y sont tels que définis dans les revendications 1, 2, 3, 4 ou 5 et L est tel que défini dans la revendication 7,
à l'exception des composés de formule II dans lesquels R1, R2, R3 et R4 représentent simultanément des atomes d'hydrogène et X est -CH₂-,
et étant exclus les composés suivants
4-(1,1-dioxo-isothiazolidin-2-yl)-3-méthyl-benzoate de méthyle,
acide 4-(1,1-dioxo-isothiazolidin-2-yl)-3-méthyl-benzoïque
4-(1,1-dioxo-1-[1,2,6]thiadiazinan-2-yl)-3-méthyl-benzoate de méthyle,
acide 4-(1,1-dioxo-1-[1,2,6]thiadiazinan-2-yl)-3-méthyl-benzoïque
et
acide 3-chloro-4-(1,1-dioxo-1-[1,2]thiazinan-2-yl)-benzoïque.

9. Composé de formule I et/ou sels pharmaceutiquement acceptables d'un tel composé, selon une ou plusieurs des revendications 1 à 6, pour utilisation en tant que médicament.

10. Utilisation d'un composé de formule I et/ou de ses sels pharmaceutiquement acceptables selon une ou plusieurs des revendications 1 à 6, pour la fabrication d'un médicament destiné au traitement ou à la prophylaxie de troubles, maladies ou maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, de l'hypertonie essentielle, de maladies du système nerveux central, de maladies qui résultent d'une hyperexcitabilité du système nerveux central, de l'épilepsie ou des crises déclenchées par le système nerveux central, de maladies du système nerveux central, en particulier d'états anxieux, de dépressions ou de psychoses, au traitement ou à la prophylaxie du diabète sucré non insulinodépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la *congestive heart failure,* de maladies inflammatoires aiguës ou chroniques, de maladies qui sont provoquées par des protozoaires, du paludisme et de la coccidiose du poulet et à l'utilisation dons des interventions chirurgicales et des transplantations d'organes, pour la conservation et le stockage de transplants pour des interventions chirurgicales, à l'utilisation dans des opérations de pontage, à l'utilisation dans la réanimation après arrêt du coeur, pour empêcher une altération tissulaire due à l'âge, pour la fabrication d'un médicament dirigé contre le vieillissement ou pour le prolongement de la vie, au traitement et à l'abaissement des effets cardiotoxiques dans la thyréotoxicose ou à la fabrication d'un agent de diagnostic.

11. Utilisation d'un composé de formule I et/ou de ses sels pharmaceutiquement acceptables selon une ou plusieurs des revendications 1 à 6, en association avec d'autres médicaments ou principes actifs pour la fabrication d'un médicament destiné au traitement ou à la prophylaxie de troubles, maladies ou maladies secondaires indirectes, aigus ou chroniques, d'organes et de tissus, qui sont provoqués par des incidents ischémiques ou par des incidents de reperfusion, au traitement ou à la prophylaxie d'arythmies, de la fibrillation ventriculaire du coeur, mettant la vie en danger, de l'infarctus du myocarde, de l'angine de poitrine, au traitement ou à la prophylaxie d'états ischémiques du coeur, d'états ischémiques du système nerveux périphérique et central ou de l'accident vasculaire cérébral ou d'états ischémiques de tissus et d'organes périphériques, au traitement ou à la prophylaxie d'états de choc, de maladies dans lesquelles la prolifération cellulaire représente une cause primaire ou secondaire, du cancer, de la dissémination de métastases, de l'hypertrophie de la prostate ou de l'hyperplasie de la prostate, de l'athérosclérose ou de troubles du métabolisme lipidique, de l'hypertension artérielle, de l'hypertonie essentielle, de maladies du système nerveux central, de maladies qui résultent d'une hyperexcitabilité du système nerveux central, telles que l'épilepsie ou des crises déclenchées par le système nerveux central, de maladies du système nerveux central, en particulier d'états anxieux, de dépressions ou de psychoses, au traitement ou à la prophylaxie du diabète sucré non insulinodépendant (DSNID) ou de complications diabétiques, de thromboses, de maladies faisant suite à un dysfonctionnement endothélial, de la claudication intermittente, au traitement ou à la prophylaxie de maladies fibreuses d'organes internes, de maladies fibreuses du foie, de maladies fibreuses des reins, de maladies fibreuses de vaisseaux et de maladies fibreuses du coeur, au traitement ou à la prophylaxie de l'insuffisance cardiaque ou de la *congestive heart failure,* de maladies inflammatoires aiguës ou chroniques, de maladies qui sont provoquées par des protozoaires, du paludisme et de la coccidiose du poulet et à l'utilisation dons des interventions chirurgicales et des transplantations d'organes, pour la conservation et le stockage de transplants pour des interventions chirurgicales, à l'utilisation dans des opérations de pontage, à l'utilisation dans la réanimation après arrêt du coeur, pour empêcher une altération tissulaire due à l'âge, pour la fabrication d'un médicament dirigé contre le vieillissement ou pour le prolongement de la vie, au traitement et à l'abaissement des effets cardiotoxiques dans la thyréotoxicose ou à la fabrication d'un agent de diagnostic.

12. Utilisation d'un composé de formule I et/ou de ses sels pharmaceutiquement acceptables selon la revendication 10, en association avec des principes actifs ou des médicaments cardiotoxiques et cytotoxiques, pour la fabrication d'un médicament à propriétés cardiotoxiques et cytotoxiques réduites.

13. Utilisation d'un composé de formule I et/ou de ses sels pharmaceutiquement acceptables, seul(s) ou en association avec d'autres principes actifs ou médicaments selon la revendication 9 et/ou la revendication 10, pour la fabrication d'un médicament destiné au traitement ou à la prophylaxie de troubles, de maladies ou de maladies secondaires indirectes, chroniques ou aigus, d'organes et de tissus, qui sont provoqués par des incidents ischémiques ou par des incidents de reperfusion.

14. Utilisation d'un composé de formule I et/ou de ses sels pharmaceutiquement acceptables, seul(s) ou en association avec d'autres principes actifs ou médicaments selon la revendication 9 et/ou la revendication 10, pour la fabrication d'un médicament destiné au traitement de la fibrillation ventriculaire du coeur, mettant la vie en danger.

15. Utilisation d'un composé de formule I et/ou de ses sels pharmaceutiquement acceptables, seul(s) ou en association avec d'autres principes actifs ou médicaments selon la revendication 9 et/ou la revendication 10, pour la fabrication d'un médicament destiné au traitement ou à la prophylaxie de la dissémination de métastases.

16. Utilisation d'un composé de formule I et/ou de ses sels pharmaceutiquement acceptables, seul(s) ou en association avec d'autres principes actifs ou médicaments selon la revendication 9 et/ou la revendication 10, pour la fabrication d'un médicament destiné au traitement ou à la prophylaxie de maladies fibreuses du coeur, de l'insuffisance cardiaque ou de la *congestive heart failure.*

17. Médicament pour l'utilisation humaine, vétérinaire ou phytosanitaire, contenant une quantité efficace d'un composé de formule I et/ou de ses sels pharmaceutiquement acceptables selon une ou plusieurs des revendications 1 à 6, conjointement avec des véhicules et additifs pharmaceutiquement acceptables.

18. Médicament pour l'utilisation humaine, vétérinaire ou phytosanitaire, contenant une quantité efficace d'un composé de formule I et/ou de ses sels pharmaceutiquement acceptables selon une ou plusieurs des revendications 1 à 6, conjointement avec des véhicules et additifs pharmaceutiquement acceptables, en association avec d'autres médicaments ou principes actifs pharmacologiques.
